# EUROPEAN PATENT APPLICATION

(11) **EP 3 772 518 A1**
(43) Date of publication of application: **10.02.2021**
(21) Application number: 19190578.5
(22) Date of filing: 07.08.2019
(51) Int. Cl.: C07K 16/00

(54) **MODIFIED HUMAN VARIABLE DOMAINS**

(71) Applicant: Merus N.V., 3584 CM Utrecht (NL)
(72) Inventor: SILVERMAN, Peter B., 3584 CM Utrecht (NL); KRAMER, Arjen, 3584 CM Utrecht (NL); BAKKER, Alexander Berthold Henrik, 3584 CM Utrecht (NL)
(74) Representative: HGF

(57) **Abstract**

The present invention provides polypeptides comprising modified human immunoglobulin heavy chain variable domains. Corresponding antibodies, variants, fragments, nucleic acids, vectors, phages, libraries, methods and kits are also provided.

## Description

### Field

The present invention provides polypeptides comprising modified human immunoglobulin heavy chain variable domains. Corresponding antibodies, variants, fragments, nucleic acids, vectors, phages, libraries, methods and kits are also provided.

### Background

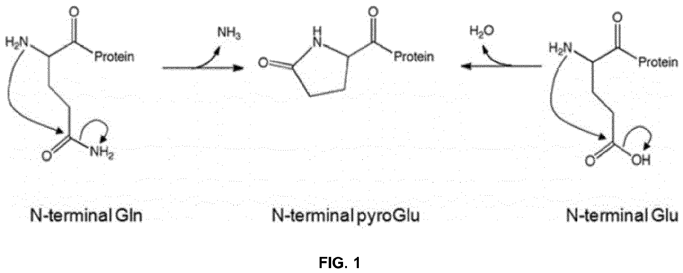

Therapeutic proteins such as antibodies contain a number of post-translational modifications, some of which have a potential unwanted impact on the protein. For example, pyroglutamate (usually abbreviated as pE, pyroE or pyroGlu) can form at the N-terminus of a polypeptide chain *in vitro* and *in vivo.* Pyroglutamate formation occurs through the rearrangement of originally synthesized glutamate or glutamine residues at this position (see Figure 1).

The first residue at the N-terminus of a human antibody heavy chain variable (VH) domain as encoded by rearrangement of a germline VH gene segment is typically either glutamine or glutamate. Both glutamine and glutamate at the N-termini of antibodies have been shown to cyclize spontaneously to pyroglutamate *in vitro.* Where pyroglutamate is formed via cyclization of glutamine, the resultant antibody becomes more acidic. Conversely, where pyroglutamate is formed via cyclization of glutamate, the resultant antibody becomes less acidic. Over time, this may lead to charge heterogeneity in antibody preparations, which may not be desirable in a variety of contexts. It may be beneficial to reduce such variability in antibody preparations.

The presence of a glutamine or glutamate at the N-terminus of a VH domain may, however, be of importance. Indeed, during secretion of antibodies from prokaryotic and eukaryotic cells, a signal peptide (SP; also known as leader peptide) is removed from the N-terminus of the immature heavy chain via cleavage between the signal peptide and the variable domain of the heavy chain. The efficiency of signal peptide cleavage depends on the sequence of the signal peptide, as well as that of the VH domain. The peptide segment recognized by signal peptide peptidase enzymes (such as SPasel) extends to the start of the mature protein (Choo *et al*., 2008). The flanking residues in the VH domain may therefore influence signal peptidase processing and contribute to non-canonical cleavage sites.

### Brief summary of the disclosure

It has been surprisingly found herein that amino acid variations can be introduced into the N-terminus of a human VH domain, whilst retaining the required affinity, specificity and/or structural interactions of the corresponding unmodified human VH domain. Advantageously, this reduces variability (e.g. charge heterogeneity) in protein preparations (e.g. antibody preparations) that include the modified human VH domain.

New antibodies, heavy chain variable regions, variants, fragments, nucleic acids, vectors, phages, libraries, methods and kits are therefore provided herein for the first time, comprising modified N-terminal residues lacking a glutamine or glutamate, while not deleteriously impacting signal peptide cleavage or protein expression. Further, described is a method of antibody generation wherein entire panels of human antibodies, heavy chain variable regions and nucleic acids that encode them can be modified to universally remove the N-terminal glutamine or glutamate to permit the display, assaying and evaluation of entire panels of such human antibodies, heavy chain variable regions ab initio, without such residue, avoiding the task of performing downstream modification upon lead candidate identification, saving time and effort, and preferably, while not deleteriously impacting signal peptide cleavage or protein expression.

Furthermore, when generating panels of heavy chains or antibodies to be screened for activity, via display technology or in functional assays, it is desirable to test such proteins as they will be in their final form to avoid variability in the results of such analysis and to avoid having to run multiple rounds of the same analyses based on variants that may be employed in downstream production. However, it is common practice to analyze heavy chains or antibodies for removal of residues that may result in post-translational modifications only after a lead, such as an antibody, has been identified, and after functional characterization has been performed. Then, specific residues can be varied through various recombinant techniques known in the art including via nucleic acid mutagenesis or through nucleic acid synthesis resulting in a new sequence encoding variations that alter residues otherwise generating potential changes to the lead during storage. Having to specifically modify each protein resulting from antibody generation, for example post-immunization and collection of B-cells of an immunized animal (transgenic or otherwise) or prior to the generation of a hybridoma or library to analyze the antibody generated, is a laborious and time-consuming process. Accordingly, a novel means of modifying an entire panel of antibodies, heavy chains or variable regions comprising a human VH domain through varying the N-terminal glutamine and glutamate has been developed herein.

A further advantage is that it is possible to use a standard vector comprising a nucleic acid encoding a signal peptide and comprising a first, or first two, amino acids of the VH domain, which first amino acid of the VH domain are not glutamine or glutamate, and wherein at least the first or first two amino acids are different from an unmodified VH domain, also referred to as a reference parental variable region, or a conventional human VH domain herein. Such vector can be used in a host cell to generate a panel of antibodies or heavy chains that comprise a modified VH domain as described herein, and further improve the robustness and efficiency of producing antibodies.

Another complexity in modifying each antibody or heavy chain produced after immunization or panel generation and prior to producing a display library is a concern that in so doing, signal peptides responsible for antibodies or heavy chains to be displayed at a cell surface can be hampered resulting in impairing the breadth and robustness of display libraries.

The fact that amino acid variation(s) can be introduced into the N-terminus of a human VH domain allows for a more efficient method to produce antibodies, as the modification can be effected during the generation of antibody panels as opposed to after one or more lead candidates have been selected.

As shown herein, elimination of the glutamine or glutamate at the N-terminus of a human VH domain and replacing such a residue with another amino acid (such as alanine) eliminates spontaneous pyroglutamate formation at the N-terminus of any protein, such as an antibody, incorporating the modified variable domain. Advantageously, successful production of Fabs and phage display libraries that incorporate the modified human VH domain demonstrate that the modified human VH domains retain the required affinity, specificity and/or structural interactions of the corresponding unmodified human VH domain. Moreover, the methodology employed, via use of universal primers, permits the generation of entire panels of human heavy chain variable regions or antibodies, which have this glutamine or glutamate at the N-terminus removed, irrespective of what V gene segment has generated the variable region, permitting uniform testing of such panels and incorporation into high throughput screening.

Several advantages may be associated with eliminating pyroglutamate formation. As stated above, the presence of pyroglutamate may alter the acidity of antibodies, which can lead to degradation of the antibody or affect shelf-life. Further, antibodies having lower pH that exceed a certain threshold level have been reported to cause pain upon infusion in patients. Eliminating pyroglutamate formation eliminates these potentially detrimental properties. Furthermore, elimination of pyroglutamate is also beneficial for regulatory purposes as, for example, the European Medical Agency guidelines require applicants of biologics to monitor several structural features including aggregation state, N- and C-termini (pyroglutamic acid at the N-terminus and lysine at the C-terminus of the heavy chain). Elimination of the N-terminal pyroglutamate will ease regulatory review by eliminating this variable for monitoring. Absence of N-terminal pyroglutamate may also result in an increase in antibody shelf life. Eliminating pyroglutamate formation also provides for greater process control and may have benefits against diseases linked to such an N-terminal glutamine or glutamate residue. The absence of N-terminal pyroglutamate also ensures a reduction in charge heterogeneity in antibodies and hence more efficient charge-based purification and separation. Addition of N-terminal modifications, and subsequent analysis might also be easier in the absence of pyroglutamate.

The inventors have also surprisingly identified that such amino acid variation(s) can bolster efficient cleavage of the signal peptide from the N-terminus of the immature human VH domain. For example, introduction of an alanine residue (optionally together with an additional second residue e.g. alanine-proline, alanine-aspartate, alanine-glutamate, alanine-threonine, alanine-valine, alanine-serine or alanine-leucine) at the N-terminus of a human VH domain may increase cleavage between the VH domain and its upstream signal peptide, thus not adversely impacting expression and secretion and preferably increase efficient expression and secretion of the modified human VH domain, as well as antibodies or antibody fragments e.g. Fabs that comprise such domains.

Several universal primers are provided herein that can be used to amplify and modify a nucleic acid that encodes a human VH domain (specifically, modifying the first (and optionally second) amino acid encoded at the N-terminus of the VH domain). Advantageously, the universal primers provided herein can, in certain combinations, amplify and modify an entire repertoire of human heavy chains generated from any functional VH gene segment present in each VH gene family of the human genome sample without bias. These universal primers are capable generating such modifications across any panel of human variable regions generated, irrespective of VH gene segment which has been recombined or rearranged to form the variable region. These universal primers are capable generating such modifications with the use of a common human heavy chain repertoire.

The invention provides a polypeptide comprising a human immunoglobulin heavy chain variable domain, wherein the variable domain comprises an N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine.

Suitably, the human immunoglobulin heavy chain variable domain may comprise an N-terminal alanine.

Suitably, the human immunoglobulin heavy chain variable domain may comprise an N-terminal sequence selected from the group consisting of: alanine-proline, alanine-aspartate, alanine-glutamate, alanine-threonine, alanine-valine, alanine-serine and alanine-leucine.

Suitably, the human immunoglobulin heavy chain variable domain can comprise the N-terminal sequence alanine-proline.

Suitably, the polypeptide may comprise a signal peptide upstream of the N-terminal amino acid of the human immunoglobulin heavy chain variable domain.

Suitably, the signal peptide can comprise the amino acid sequence AQPAMA (SEQ ID NO: 5).

The invention also provides an antibody, antibody variant or antibody fragment comprising a polypeptide as described herein.

The invention further provides a nucleic acid encoding a polypeptide, antibody, antibody variant or antibody fragment as described herein.

Moreover, the invention provides a vector comprising a nucleic acid as described herein.

Suitably, the vector can be a phagemid or a plasmid.

Additionally, the invention provides a phage comprising a nucleic acid as described herein.

Also provided herein is a library comprising at least about 10⁶ distinct nucleic acids, vectors, or phages as described herein.

The invention provides a method of simultaneously amplifying and modifying a nucleic acid that encodes a human immunoglobulin heavy chain variable domain, the method comprising:
(a) providing a nucleic acid that encodes a human immunoglobulin heavy chain variable domain; and
(b) performing a polymerase chain reaction with at least one 5' primer, at least one 3' primer and the nucleic acid to generate an amplified nucleic acid,
wherein the at least one 5' primer comprises a nucleic acid with a modification site that introduces a modification in the amplified nucleic acid such that the amplified nucleic acid encodes a human immunoglobulin heavy chain variable domain comprising an N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine.

Suitably, each amplified nucleic acid may encode a human immunoglobulin heavy chain variable domain comprising an N-terminal alanine.

Suitably, the at least one 5' primer could comprise a nucleic acid that introduces at least two modifications in each amplified nucleic acid such that each amplified nucleic acid encodes a human immunoglobulin heavy chain variable domain comprising an N-terminal sequence selected from the group consisting of: alanine-proline, alanine-aspartate, alanine-glutamate, alanine-threonine, alanine-valine, alanine-serine and alanine-leucine.

Suitably, each of the amplified nucleic acid may encode a human immunoglobulin heavy chain variable domain comprising the N-terminal sequence alanine-proline.

Suitably, the at least one 5' primer could encode a signal peptide or portion of a signal peptide upstream of the modification site.

Suitably, the signal peptide can comprise the amino acid sequence AQPAMA (SEQ ID NO:5).

Suitably, the nucleic acid(s) in step (a) may be cDNA.

Suitably, the method could comprise a prior step of extracting nucleic acids from B cells of an animal and generating cDNA from the nucleic acids to generate the nucleic acid(s) provided in step (a).

Suitably, the animal could have been immunized with an antigen of interest, and the nucleic acids from the B cells may encode heavy chains having specificity and affinity for the antigen of interest.

Suitably, the animal can be a transgenic murine animal comprising a human immunoglobulin heavy chain locus.

Suitably, the animal may be a transgenic murine animal comprising a common light chain.

Suitably, all nucleic acids that encode a human immunoglobulin heavy chain variable domain from the B cells can be modified to encode:
- human immunoglobulin heavy chain variable domains comprising an N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine; or
- human immunoglobulin heavy chain variable domains comprising an N-terminal sequence selected from the group consisting of: alanine-proline, alanine-aspartate, alanine-glutamate, alanine-threonine, alanine-valine, alanine-serine and alanine-leucine.

Suitably, step (a) may comprise providing a plurality of distinct nucleic acids encoded by at least one recombined human gene segment selected from each of the following human gene families: IGHV1, IGHV2, IGHV3, IGHV4, IGHV5, IGHV6 and IGHV7.

Suitably, the method may comprise:
(a) amplifying and modifying a nucleic acid encoded by an IGHV1 family gene using a 5' primer that is selected from 1308AP, 1308AP2, 2020AP2, 2018AP or 2018AP2; and/or
(b) amplifying and modifying a nucleic acid encoded by an IGHV2 family gene using a 5' primer that is selected from 1310AP2, 1310AP3, 1310AP4, or1310AP5; and/or
(c) amplifying and modifying a nucleic acid encoded by an IGHV3 family gene using a 5' primer that is selected from 0508AP, 0508AP2, 2018AP, 2018AP2, 2021AP, 2021AP2, 2021AP3, 2021AP4, or 2021AP5; and/or
(d) amplifying and modifying a nucleic acid encoded by an IGHV4 family gene using a 5' primer that is 1312AP; and/or
(e) amplifying and modifying a nucleic acid encoded by an IGHV5 family gene using a 5' primer that is selected from 1313AP, or 1313AP2;
(f) amplifying and modifying a nucleic acid encoded by an IGHV6 family gene using a 5' primer that is selected from 1310AP2, 1310AP3, 1310AP4, 1310AP5 or 1312AP2; and/or
(g) amplifying and modifying a nucleic acid encoded by an IGHV7 family gene using a 5' primer that is selected from 1314AP, or 1314AP2.

Suitably, the method could comprise:
(a) amplifying and modifying a nucleic acid encoded by an IGHV1 family gene using a 5' primer that is selected from 1308AP2, 2020AP2, or 2018AP2; and/or
(b) amplifying and modifying a nucleic acid encoded by an IGHV2 family gene using a 5' primer that is 1310AP5; and/or
(c) amplifying and modifying a nucleic acid encoded by an IGHV3 family gene using a 5' primer that is selected from 0508AP; 2021AP2 or 2018AP2]; and/or
(d) amplifying and modifying a nucleic acid encoded by an IGHV4 family gene using a 5' primer that is selected from 1312AP2 or 2019AP2; and/or
(e) amplifying and modifying a nucleic acid encoded by an IGHV5 family gene using a 5' primer that is 1313AP2;
(f) amplifying and modifying a nucleic acid encoded by an IGHV6 family gene using a 5' primer that is selected 1310AP2, 1310AP3, 1310AP4, 1310AP5 or 1312AP2; and/or
(g) amplifying and modifying a nucleic acid encoded by an IGHV7 family gene using a 5' primer that is 1314AP2.

Suitably, the modified human immunoglobulin heavy chain variable domains could be subjected to frequency analysis for lead identification.

Suitably, the method may further comprise introducing each amplified and modified nucleic acid into a vector.

Suitably, the vector could be a phagemid or a plasmid.

Suitably, the method may further comprise transforming or transfecting each vector into a cell to generate a library.

Suitably, the cell can be a phage competent cell.

Suitably, the modified human immunoglobulin heavy chain variable domain could be integrated into a phage for screening for binding specificity and/or affinity.

The invention further provides a 5' primer for amplifying and modifying any nucleic acid that encodes a human immunoglobulin heavy chain variable domain selected from one or more of the following human VH gene families: IGHV1, IGHV2, IGHV3, IGHV4, IGHV5, IGHV6 and IGHV7, wherein the primer comprises a modification site that introduces a modification in the amplified nucleic acid such that the amplified nucleic acid encodes a human immunoglobulin heavy chain variable domain comprising an N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine.

Suitably, the modification site could be such that the amplified nucleic acid encodes a human immunoglobulin heavy chain variable domain comprising an N-terminal alanine.

Suitably, the modification site could be such that the amplified nucleic acid encodes a human immunoglobulin heavy chain variable domain comprising an N-terminal sequence selected from the group consisting of: alanine-proline, alanine-aspartate, alanine-glutamate, alanine-threonine, alanine-valine, alanine-serine and alanine-leucine.

Suitably, the modification site could be such that the amplified nucleic acid encodes a human immunoglobulin heavy chain variable domain comprising an N-terminal alanine-proline.

Suitably, the primer may encode a signal peptide or portion of a signal peptide upstream of the modification site.

Finally, the invention provides a kit comprising at least one 5' primer selected from each of the following groups:
(a) 1308AP, 1308AP2, 2020AP2, 2018AP or 2018AP2;
(b) 1310AP2, 1310AP3, 1310AP4 or 1310AP5; a
(c) 0508AP, 0508AP2, 2018AP, 2018AP2, 2021AP, 2021AP2, 2021AP3, 2021AP4, or 2021AP5;
(d) 1312AP2;
(e) 1313AP or1313AP2;
(f) 1310AP2, 1310AP3, 1310AP4, 1310AP5, or 1312AP2;
   and
(g) 1314AP2 or 1314AP.

Suitably, the kit may comprise at least one 5' primer selected from each of the following groups:
(a) 1308AP2, 2020AP2, or 2018AP2;
(b) 1310AP5;
(c) 0508AP, 2021AP2, or 2018AP2;
(d) 1312AP2, or 2019AP2;
(e) 1313AP2;
(f) 1310AP2, 1310AP3, 1310AP4, 1310AP5, or 1312AP2; and
(g) 1314AP2.

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

Various aspects of the invention are described in further detail below.

### Brief description of the drawings

Embodiments of the invention are further described hereinafter with reference to the accompanying drawings, in which:
Figure 1 shows pyroglutamic acid (pyroGlu) formation at the N-terminus of proteins.
Figure 2 shows the results of SP cleavage prediction analysis using SignalP. For each of 360 analysed sequences, the D score is given, as well as the average (avg.) D score per amino acid at VH position 1. Data for sequences with a prokaryotic (P) SP are given on the left; data for sequences with a eukaryotic (E) SP are given on the right. To the right side of each data set, column 'nat. freq. (%)' lists the amino acid frequencies at position 1 for a panel of Gram-negative and eukaryotic SP-containing proteins [Choo and Ranganathan, 2008].
Figure 3 shows the results of SP cleavage prediction analysis using SignalP for 1170 sequences. For 1170 analysed sequences, the D score is given, as well as the average (avg.) D score per amino acid at VH position 2. Data for sequences with a prokaryotic (P) SP are given on the left; data for sequences with a eukaryotic (E) SP are given on the right. To the right side of each data set, column 'nat. freq. (%)' lists the amino acid frequencies at position 2 for a panel of Gram-negative and eukaryotic SP-containing proteins [Choo and Ranganathan, 2008].

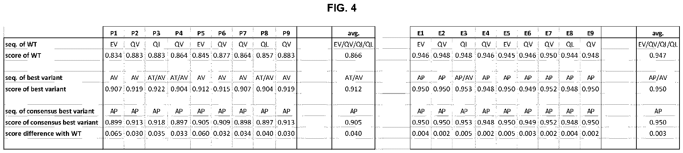

Figure 4 shows the results of SP cleavage prediction analysis using SignalP. For each of the 18 combinations of SP and start of VH, D scores are given for a subset of the 1170 sequences with variations at position 1 and 2. Data for sequences with a prokaryotic (P) SP are given on the left; data for sequences with a eukaryotic (E) SP are given on the right. For both SP's, the average (avg.) D score is given as well. The upper rows show results for the WT sequences. The middle rows show results for the best variants, i.e. the sequences with the highest D scores. The lower rows show results for the consensus of the best variants, i.e. the sequences with D scores that are higher than those of the WT sequences, both when combined with the bacterial SP and when combined with the eukaryotic SP; the D score differences compared to WT are given as well (last row).
Figure 5 shows an alignment of new 5' AP primers (first two VH codons bold and underlined).
Figure 6 shows a protein translation of newly designed 5' primers annealing to the start of VH gene segments expressed by two Merus Mouse (MeMo®) lines. The first two residues of the VH regions, which were changed to AP, are bold and underlined.
Figure 7 shows the amplification efficiency for AP 5' primers (comparison of PCR product yields on agarose gel).
Figure 8 shows an alignment of new AP and AP2 primers.
Figure 9 shows the amplification efficiency for AP2 5' primers (comparison of PCR product yields on agarose gel).
Figure 10 shows the amplification efficiency for all new primers (AP and AP2) tested in parallel.
Figure 11 shows the amplification efficiency for 5 different variants of the 1310AP primer, together with five different variants of the 2021AP primer.

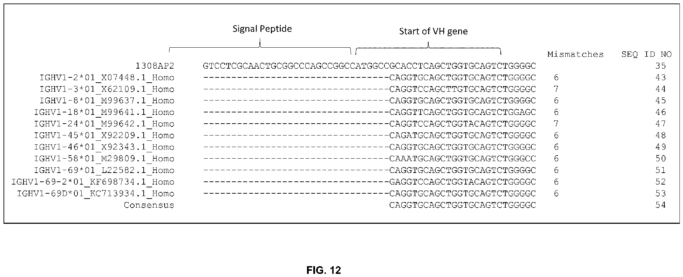

Figure 12 shows an alignment of new primer 1308AP2 with all functional IGHV1 sequences.

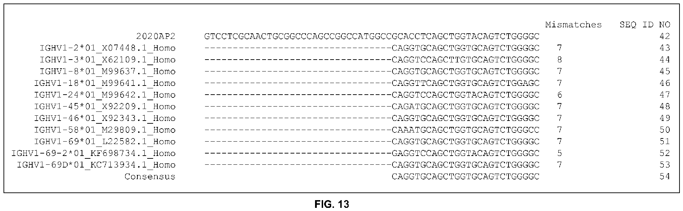

Figure 13 shows an alignment of new primer 2020AP2 with all functional IGHV1 sequences.

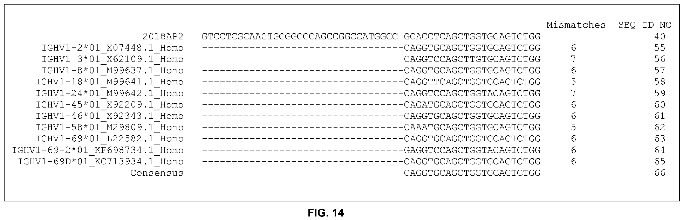

Figure 14 shows an alignment of new primer 2018AP2 with all functional IGHV1 sequences.

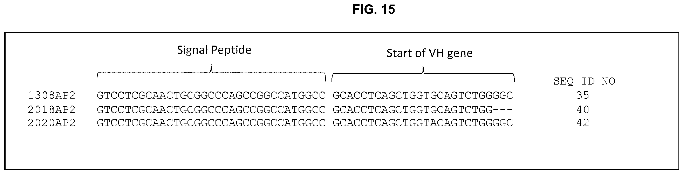

Figure 15 shows an alignment of new primers 1308AP2, 2018AP2 and 2020AP2 for IGHV1.

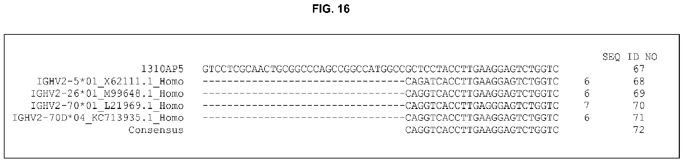

Figure 16 shows an alignment of new primer 1310AP5 with all functional IGHV2 sequences.

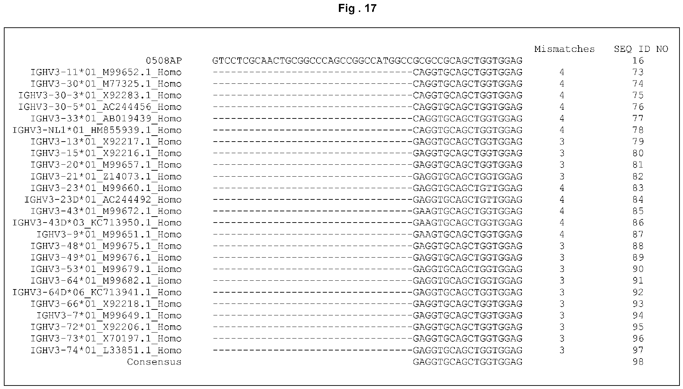

Figure 17 shows an alignment of new primer 0508AP with all functional IGHV3 sequences.

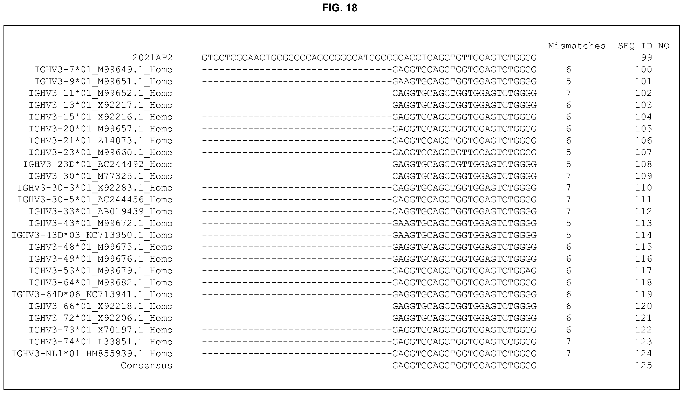

Figure 18 shows an alignment of new primer 2021AP2 with all functional IGHV3 sequences.

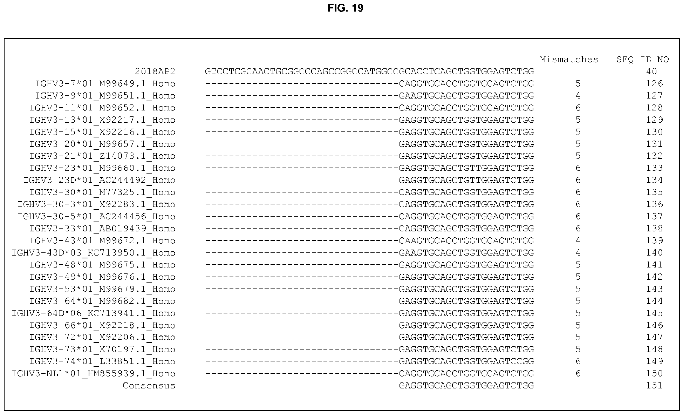

Figure 19 shows an alignment of new primer 2018AP2 with all functional IGHV3 sequences.

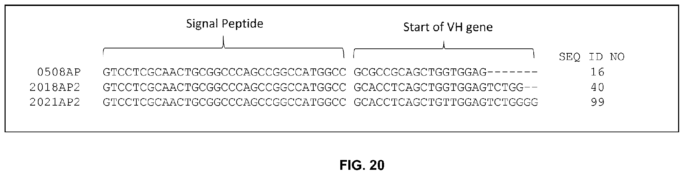

Figure 20 shows an alignment of three new primer specific to IGHV3.

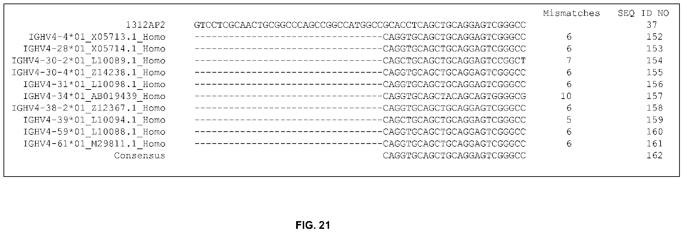

Figure 21 shows an alignment of new primer 1312AP2 with all functional IGHV4 sequences.

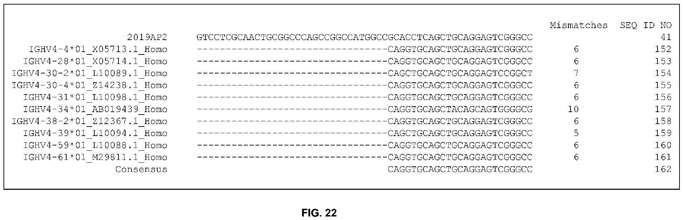

Figure 22 shows an alignment of new primer 2019AP2 with all functional IGHV4 sequences 2019AP2.

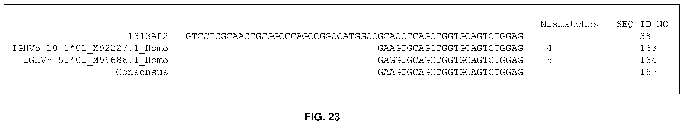

Figure 23 shows an alignment of new primer 1313AP2 with all functional IGHV5 sequences.

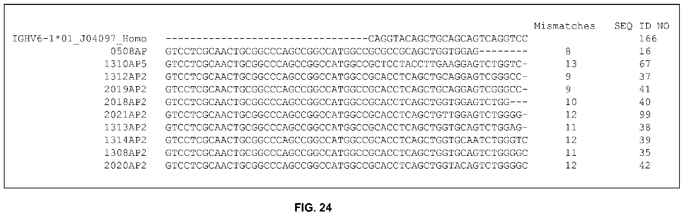

Figure 24 shows an alignment of new primers with IGHV6-1.

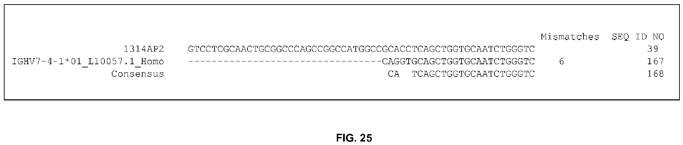

Figure 25 shows an alignment of new primer 1314AP2 with functional IGHV7 gene segment.

The figures provided herein show sequence alignments of specific VH gene segments within each family, with their corresponding primers. It would clear to a person of ordinary skill in the art that VH gene segments may vary in sequence due to allelic variation and corresponding primers to different VH gene segment sequences within each family are also encompassed by the invention described herein

The patent, scientific and technical literature referred to herein establish knowledge that was available to those skilled in the art at the time of filing. The entire disclosures of the issued patents, published and pending patent applications, and other publications that are cited herein are hereby incorporated by reference to the same extent as if each was specifically and individually indicated to be incorporated by reference. In the case of any inconsistencies, the present disclosure will prevail.

Various aspects of the invention are described in further detail below.

### Detailed description

The present invention provides polypeptides comprising modified human immunoglobulin heavy chain variable (VH) domains.

The terms "immunoglobulin heavy chain variable domain" and "VH domain" are used interchangeably herein. The terms are generally used herein to refer to human VH domains (unless the context specifically indicates otherwise).

The terms "peptide", "protein" and "polypeptide" are used interchangeably herein. The N-terminus of a protein sequence (also known as the amino-terminus, NH2-terminus, N-terminal end or amine-terminus) is the start of that protein sequence. By convention, peptide sequences are written N-terminus to C-terminus (from left to right). The C-terminus (also known as the carboxyl-terminus, carboxy-terminus, C-terminal tail, C-terminal end, or COOH-terminus) is the end of an amino acid chain (protein or polypeptide), terminated by a free carboxyl group (-COOH).

The modified human variable domain (also referred to as a modified VH domain herein) described herein comprises an amino acid modification compared to a conventional human VH domain, namely that the N-terminal amino acid of the modified VH domain is selected from the group consisting of: alanine, arginine, asparagine, aspartate, glycine, histidine, isoleucine, leucine, lysine, methionine, phenylalanine, proline, serine, threonine, tryptophan, tyrosine and valine. This is distinct from a conventional human VH domain, also referred to herein as an unmodified VH domain, as conventional human VH domains have a glutamine or glutamate residue at the N-terminus.

As used herein, the "N-terminus" of a VH domain (or the "N-terminal amino acid" of a VH domain) refers to the start of the VH domain amino acid sequence (i.e. the first amino acid (from left to right) of the VH domain), irrespective of what other peptide domains and sequences may be present within the polypeptide. For the avoidance of doubt, the "N-terminus" of a VH domain (or the "N-terminal amino acid" of a VH domain) therefore refers to the first amino acid of the mature VH domain amino acid sequence and does not take into account any upstream amino acids that may be present in the polypeptide as part of e.g. a signal peptide sequence. Therefore the "N-terminus" of a VH domain (or the "N-terminal amino acid" of a VH domain) may not actually be at the start of the polypeptide chain (it may have other amino acid residue(s) upstream of it). In other words, for polypeptides that comprise a signal peptide directly upstream and adjacent to a VH domain amino acid sequence, the "N-terminus" of the VH domain refers to the first amino acid of the VH domain (i.e. the first amino acid after the signal peptide sequence). As a non-limiting example, when a polypeptide comprises a signal peptide (MKYLLPTAAAGLLLLAAQPAMA (SEQ ID NO:1) + VH domain (QVQLVQSG (SEQ ID NO:2).....(as per IGHV1-3*01_X62109.1_Homo)) as shown in sequence MKYLLPTAAAGLLLLAAQPAMAQVQLVQSG (SEQ ID NO:3) ....., the N-terminal amino acid of the VH domain is underlined.

VH domains are made up of four framework regions and three hypervariable regions (also known as CDRs), having the arrangement FR1-CDR1-FR2-CDR2-FR3-CDR3-FR4 (N-terminus to C-terminus). The framework regions make up about 85% of the variable region and act as a scaffold for the CDRs of the VH domain. The framework regions have less variability in their amino acid sequences compared to the CDRs. The first amino acid of the FR1 region is also the N-terminal amino acid of the VH domain. The Kabat numbering scheme is extensively used for the numbering of residues in antibody sequences. (Kabat, E.A. et al., NIH Publication No. 91-3242 (1991)).

Although the N-terminal residue glutamine or glutamate of a human VH domain is thought to play an important (or critical) role in antigen affinity, antigen specificity and/or structural interactions of an antibody, it has now surprisingly been found that amino acid variations at the position can be tolerated. Advantageously, this allows modification of the glutamine or glutamate with another (preferable) amino acid, such as one of alanine, arginine, asparagine, aspartate, glycine, histidine, isoleucine, leucine, lysine, methionine, phenylalanine, proline, serine, threonine, tryptophan, tyrosine or valine. Modification of the N-terminal amino acid of a human VH domain in this way eliminates the formation of pyroglutamate (and thus avoids the potential deleterious effects associated with pyroglutamate formation which are discussed in detail elsewhere herein).

Amino acids may be grouped according to their biochemical properties (e.g. charge, hydrophobicity, size etc). For example, acidic residues include aspartate and glutamate, Examples of non-acidic residues with polar side chains include asparagine and glutamine. In one example, therefore, the N-terminal residue glutamine or glutamate of a human VH domain is replaced with an acidic or polar residue, such as aspartate or asparagine. These amino acids have similar biochemical properties to glutamine or glutamate and thus may be useful choices, as such changes retain similar biochemical properties whilst removing the potential downstream variability in heavy chain variable regions over time that may come from glutamine or glutamate to pyroglutamate transition.

The potential effect of each amino acid variation (i.e. N-terminal alanine, arginine, asparagine, aspartate, glycine, histidine, isoleucine, leucine, lysine, methionine, phenylalanine, proline, serine, threonine, tryptophan, tyrosine or valine) on recognition of the signal peptide cleavage site upstream of a human VH domain has been analysed herein. It has been found that modification of the N-terminal glutamate or glutamine residue in human VH domains with alanine is particularly useful as it eliminates pyroglutamate formation and also maintains (e.g. improves) recognition of the signal peptide cleavage site in prokaryotes and eukaryotes. Other amino acids that eliminate pyroglutamate formation whilst maintaining relative recognition of the signal peptide cleavage site in prokaryotes based on in silico review of average D score, are glycine, methionine, asparagine, serine, threonine, valine and tyrosine. Other amino acids that eliminate pyroglutamate formation whilst maintaining relative recognition of the signal peptide cleavage site in eukaryotes, based on an in silico review of average D score, include phenylalanine, isoleucine, leucine, valine and tryptophan. Each of these amino acids is therefore also useful for the modification of the N-terminal glutamate or glutamine residue. Evaluating the elimination of pyroglutamate formation whilst maintaining relative recognition of the signal peptide cleavage site in both prokaryotes and eukaryotes based on unmodified residue frequency indicates that the preferred residues are alanine, aspartic acid and serine.

Alanine is an aliphatic residue. Accordingly, in another example, the N-terminal residue glutamine or glutamate of a human VH domain is replaced with an aliphatic residue such as alanine, glycine, valine, leucine or isoleucine.

The effects of replacing the second amino acid at the N-terminus of the VH domain with another (preferential) amino acid have also been investigated herein. For the avoidance of doubt, "second amino acid at the N-terminus of the VH domain" refers to the amino acid directly adjacent (in a N-terminal to C-terminal direction) to the N-terminal amino acid of the VH domain (in other words, the amino acid at position two in the VH domain amino acid sequence, wherein the N-terminal amino acid (such as the Q or E in unmodified human VH domains) is at position one).

Surprisingly, it has been found that changing the second amino acid at the N-terminus of the VH domain (in a N-terminal to C-terminal direction) to one of proline, aspartate, glutamate, threonine, valine, serine or leucine promotes cleavage of the signal peptide cleavage site upstream of the VH domain based on the unmodified frequency of such residues in mature proteins of Gram-negative bacteria having a signal peptide (Choo and Ranganathan, 2008). This is particularly the case when proline is selected as the second amino acid at the N-terminus of the VH domain.

Accordingly, the modified human VH domain described herein may comprise a first N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine; and a second amino acid selected from the group consisting of proline, aspartate, glutamate, serine, threonine, valine or leucine. In one specific example, the second amino acid at the N-terminus of the VH domain is selected to be proline, with preferred first and second position comprising "alanine-proline" or "AP". Preferably, a cysteine residue here is avoided as it introduces of a highly reactive group at the N-terminus, which may cause development liabilities in manufacture and storage.

As shown in the data presented below, particularly useful combinations of amino acids at the N-terminal of the modified VH domain are alanine-proline, alanine-aspartate, alanine-glutamate, alanine-serine, alanine-threonine, alanine-valine and alanine-leucine. As used herein the formatting used for "alanine-proline" or "AP" etc. refers to the two adjacent amino acids at the N-terminal end of the modified human VH domain (i.e. the "first-second" amino acids at the N-terminus of the VH domain (in a N-terminal to C-terminal direction)).

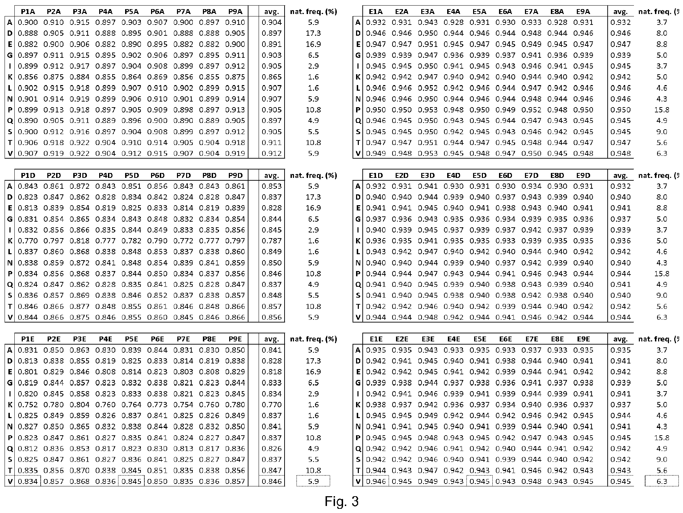

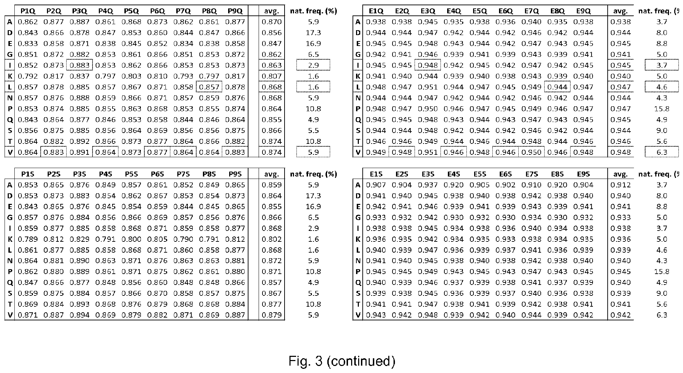

Alternative preferred combinations were deduced from the SP cleavage prediction analysis using signalP. Based on the data in Figure 3, other preferred combinations include each of glycine-proline, glycine-aspartate, glycine-glutamate, glycine-serine, glycine-threonine, glycine-leucine, glycine-valine, methionine-proline, methionine-aspartate, methionine-glutamate, methionine-serine, methionine-threonine, methionine-leucine, methionine-valine, asparagine-proline, asparagine-aspartate, asparagine-glutamate, asparagine-serine, asparagine-threonine, asparagine-leucine, asparagine-valine, serine-proline, serine-aspartate, serine-glutamate, serine-serine, serine-threonine, serine-leucine, serine-valine, threonine-proline, threonine-aspartate, threonine-glutamate, threonine-serine, threonine-threonine, threonine-leucine, threonine-valine, valine-proline, valine-aspartate, valine-glutamate, valine-serine, valine-threonine, valine-leucine, valine-valine, tyrosine-proline, tyrosine-aspartate, tyrosine-glutamate, tyrosine-serine, tyrosine-leucine, tyrosine-valine, and tyrosine-threonine.

The effects of modifying the first two amino acids at the N-terminus of a human VH domain to alanine-proline have been investigated herein. This combination was found to be particularly advantageous for promoting signal peptide cleavage efficiency. The invention described herein is therefore particularly advantageous when used with a modified human VH domain that is expressed together with an upstream signal peptide in a cell. In this context, "upstream" refers to the positioning of the signal peptide in the polypeptide relative to the modified VH domain. In other words, when looking at the polypeptide as a whole, the signal peptide is upstream of the modified VH domain when it is closer to the N-terminus of the polypeptide than the amino acid sequence of the modified VH domain.

Several different signal peptides are known to those of ordinary skill in the art. As used herein, the term "signal peptide" refers to a leader sequence ensuring entry into the secretory pathway. A signal peptide is a relatively short peptide located at the N-terminus of secretory proteins which direct the protein to the lumen of endoplasmic reticulum for subsequent export from the cell. For example, in eukaryotes, a signal peptide that contains 5-30 amino acids present at the N-terminus of nascent proteins is recognized by the signal recognition particle (SRP) in the cytosol while the protein is still being synthesized on the ribosome. The SRP then delivers the SRP-ribosome-nascent chain (SRP-RNC) complex to the SRP-receptor (SR) in the endoplasmic reticulum (ER) membrane. GTP-dependent mechanisms then deliver the RNC complex to a membrane-bound translocon which allows translocation of the growing polypeptide chain into the lumen of the ER. After crossing the ER membrane, the signal peptide is cleaved off by a signal peptide peptidase (SPP). Several signal peptides suitable for protein expression in eukaryotic cells are known to those of ordinary skill in the art. N-terminus of immunoglobulin heavy and light chains have native signal peptide or leader sequences (Nucl. Acids Res. (2005), 33, D256-D261). Other signal peptide sequences are well known in the art, as listed in Nucleic Acid Research (1984) 12, 5145 - 5164. Examples of signal peptides of membrane proteins and secretory proteins include those derived from *Saccharomyces cerevisiae* or yeast viruses that are used in conventional membrane and secretory protein expression systems in yeast include secretory signal peptides derived from α-factor, α-factor receptor, preprotoxin, SUC2 proteins and PHO5 proteins, BGL2 proteins, and AGA2 proteins. Computer programs that predict the sequences of secretory signal peptides have been provided. SignalP (http://www.cbs.dtu.dk/services/SignaIP/), PSORT (http://psort.nibb.ac.jp/), and Phobius (http://phobius.cgb.ki.se/). Use of these computer programs permit sequence prediction of the secretory signal.

A particular example of a signal peptide that can be used for VH domain expression in eukaryotic cells (which is used in the examples section below) is MGWSCIILFLVLLLAQPAMA (SEQ ID NO:4). It is noted that this is a non-limiting example as SRP selectively bind signal peptides based on their common features, despite their variability in primary sequence. Accordingly, other appropriate signal peptides may also be used herein.

Most proteins that are transported to the extracytoplasmic environment in bacteria utilize the so-called Sec pathway for targeting. This pathway is initiated when a signal peptide on a newly synthesized precursor protein is recognized by SecA, a protein that occurs only in prokaryotes and organelles of prokaryotic origin, such as mitochondria.

A particular example of a signal peptide that can be used for VH domain expression in prokaryotic cells (which is used in the examples section below) is MKYLLPTAAAGLLLLAAQPAMA (SEQ ID NO:1). It is noted that this is a non-limiting example as SecA selectively binds signal peptides based on their common features, despite their variability in primary sequence. Accordingly, other appropriate signal peptides may also be used herein. Representative signal sequences include signal sequences from PelB, OmpA, PhoA, endoxylanase and StlI (Appl. Microbiol. Biotechnol (2004) 64:625-635).

It is noted that the two (non-limiting) signal peptides used in the examples section below both have the sequence AQPAMA (SEQ ID NO:5) at the C-terminal end of the signal peptide (in other words, the amino acid sequence immediately upstream of the N-terminus of the VH domain is ...AQPAMA (SEQ ID NO:5)). Accordingly, in one example, the signal peptide comprises the amino acid sequence AQPAMA (SEQ ID NO:5).

As stated above, the efficiency of signal peptide cleavage depends on the sequence of the signal peptide at the signal peptide cleavage site, as well as that of the VH. The residues of the VH domain and signal peptide that flank the signal peptide cleavage site may therefore influence signal peptidase processing and contribute to non-canonical cleavage sites. Preferably, these residues are therefore **AQPAMA**A (SEQ ID NO:6) or **AQPAMA**AP (SEQ ID NO:7) (with the sequence at the C-terminal end of the signal peptide in bold, and the N-terminal amino acid of the modified human VH domain underlined).

In a further example, specifically for expression in prokaryotic host cells, the amino acid residues of the VH domain and signal peptide that flank the signal peptide cleavage site may therefore be MKYLLPTAAAGLLLLA**AQPAMA**A (SEQ ID NO:8) or MKYLLPTAAAGLLLLA**AQPAMA**AP (SEQ ID NO:9) (with the sequence at the C-terminal end of the signal peptide in bold, and the N-terminal amino acid of the modified human VH domain underlined).

In a further example, specifically for expression in eukaryotic host cells, the amino acid residues of the VH domain and signal peptide that flank the signal peptide cleavage site may therefore be MGWSCIILFLVLLL**AQPAMA**A (SEQ ID NO:10) or MGWSCIILFLVLLLA**QPAMA**AP (SEQ ID NO:11) (with the sequence at the C-terminal end of the signal peptide in bold, and the N-terminal amino acid of the modified human VH domain underlined).

The polypeptides described herein comprise a modified VH domain. The polypeptide may be any protein that includes the modified VH domain described herein. For example, it may be an antibody, an antibody variant or an antibody fragment that includes the modified VH domain described herein.

### Antibodies, antibody variants and antibody fragments

An "antibody" is a proteinaceous molecule belonging to the immunoglobulin class of proteins, containing one or more domains that bind an epitope on an antigen, where such domains are derived from or share sequence homology with the variable region of an antibody. Antibody binding has different qualities including specificity and affinity. The specificity determines which antigen or epitope thereof is specifically bound by the binding domain. The affinity is a measure for the strength of binding to a particular antigen or epitope. It is convenient to note here that the 'specificity' of an antibody refers to its selectivity for a particular antigen, whereas 'affinity' refers to the strength of the interaction between the antibody's antigen binding site and the epitope it binds. Thus, the "binding specificity" as used herein refers to the ability of an individual antibody binding site to react with an antigenic determinant. Typically, the binding site of the antibody of the invention is located in the Fab portions and is constructed from the hypervariable regions of the heavy and light chains.

A used herein, an "antibody fragment" refers to a proteinaceous moiety comprising a functional part of an antibody (in this case at least the modified VH domain described herein). The antibody fragment can be any binding agent, including, but not limited to, single chain Fvs, single chain or Tandem diabodies (TandAb®), VHHs, Anticalins®, Nanobodies®, a BiTE®, a Fab, ankyrin repeat proteins or DARPINs®, Avimers®, a DART, a TCR-like antibody, Adnectins®, Affilins®, Trans-bodies®, Affibodies®, a TrimerX®, MicroProteins, Fynomers®, Centyrins® or a KALBITOR®.

"Affinity" is the strength of the interaction between a single antigen-binding site and its antigen. A single antigen-binding site of an antibody of the invention for an antigen may be expressed in terms of the equilibrium dissociation constant (Kd), also known as affinity constant. Typically, antibodies for therapeutic applications may have affinities with Kd values in the micromolar (10⁻⁶M; low affinity) to picomolar (10⁻¹² M; high affinity) range.
An "antigen" is a molecule capable of inducing an immune response (to produce an antibody) in a host organism and/or being targeted by an antibody. At the molecular level, an antigen is characterized by its ability to be bound by the antigen-binding site of an antibody. Also mixtures of antigens can be regarded as an 'antigen', i.e. The skilled person would appreciate that sometimes a lysate of tumor cells, or viral particles may be indicated as 'antigen' whereas such tumor cell lysate or viral particle preparation exists of many antigenic determinants. An antigen comprises at least one, but often more, epitopes.

An "epitope" or "antigenic determinant" is a site on an antigen to which an immunoglobulin or antibody specifically binds. Epitopes can be formed from contiguous amino acids or non-contiguous amino acids juxtaposed by tertiary folding of a protein (so-called linear and conformational epitopes, respectively). Epitopes formed from contiguous, linear amino acids are typically retained on exposure to denaturing solvents, whereas epitopes formed by tertiary folding, conformation are typically lost on treatment with denaturing solvents. An epitope may typically include 3, 4, 5, 6, 7, 8, 9, 10, 11, 12, 13, 14 or 15 amino acids in a unique spatial conformation.

The term "heavy chain" or "immunoglobulin heavy chain" includes an immunoglobulin heavy chain constant region sequence from any organism, and unless otherwise specified includes a heavy chain variable domain (VH). Heavy chain variable domains include three heavy chain CDRs and four frame work (FR) regions, unless otherwise specified. Fragments of heavy chains include CDRs and FRs, and combinations thereof. A typical heavy chain has, following the variable domain (from N-terminal to C-terminal), a CH1 domain, a hinge, a CH2 domain, and a CH3 domain. A functional fragment of a heavy chain includes a fragment that is capable of specifically recognizing an antigen and that comprises at least one CDR.

The term "light chain" includes an immunoglobulin light chain variable domain, or V_{L} (or functional fragment thereof); and an immunoglobulin constant domain, or C_{L} (or functional fragment thereof) sequence from any organism. Unless otherwise specified, the term light chain may include a light chain selected from a human kappa, lambda, and a combination thereof. Light chain variable (V_{L}) domains typically include three light chain CDRs and four FR regions, unless otherwise specified. Generally, a full-length light chain includes, from N-terminus to C-terminus, a V_{L} domain that includes FR1-CDR1-FR2-CDR2-FR3-CDR3-FR4 and a light chain constant domain. Light chains that can be used with this invention include those, e.g., that do not selectively bind an epitope selectively bound by the heavy chains.

Suitable light chains for use in an antibody of the invention include a common light chain (cLC), such as those that can be identified by screening for the most commonly employed light chains in existing antibody libraries (wet libraries or in silico), where the light chains do not substantially interfere with the affinity and/or selectivity of the epitope-binding domains of the heavy chains, but are also suitable to pair with an array of heavy chains. For example, a suitable light chain includes one from a transgenic animal, such as MeMo® having the common light chain integrated into its genome and which can be used to generate large panels of common light chain antibodies having diversity at the heavy chain and capable of specifically binding an antigen upon exposure to said antigen.

The term "common light chain" refers to light chains which may be identical or have some amino acid sequence differences while the binding specificity of an antibody of the invention is not affected, i.e. the differences do not materially influence the formation of functional binding regions. It is for instance possible within the scope of the definition of common chains as used herein, to prepare or find variable chains that are not identical but still functionally equivalent, e.g., by introducing and testing conservative amino acid changes, changes of amino acids in regions that do not or only partly contribute to binding specificity when paired with a cognate chain, and the like. Such variants are thus also capable of binding different cognate chains and forming functional antigen binding domains. The term 'common light chain' as used herein thus refers to light chains which may be identical or have some amino acid sequence differences while retaining the binding specificity of the resulting antibody after pairing with a heavy chain. A combination of a certain common light chain and such functionally equivalent variants is encompassed within the term "common light chain". Reference is made to WO 2004/009618 and WO2009/157771 for a detailed description of the use of common light chains.

A "Fab" means a binding domain comprising a variable region, typically a binding domain comprising a paired heavy chain variable domain and light chain variable domain. A Fab may comprise constant region domains, including a CH1 and a VH domain paired with a constant light domain (CL) and VL domain. Such pairing may take place, for example, as covalent linkage via a disulfide bridge at the CH1 and CL domains.

A "single-chain variable fragment" (scFv) means a binding domain comprising a VH domain and a VL domain which are connected via a linker, for example a peptide linker, for example from about 10 to about 25 amino acids in length. Herein, the term "connected" refers to domains which are joined to each other by way of their primary amino acid sequence. For example, a base antibody portion may be connected to an additional binding domain (or an additional binding domain to an additional binding domain) via a linker. Similarly, a CH1 domain may be connected to a variable heavy region and a CL domain may be connected to a variable light region. "Pairing" then refers to interactions between the polypeptides of the invention such that they may multimerize. For example, an additional binding domain may comprise a heavy chain region (CH1-VH) paired to a light chain region (CL-VL), where the CH1 (of the heavy chain region) and the CL (of the light chain region) pair typically by the formation of a disulphide bond. Domains of antibody chains or polypeptides, such as a mixed binding domain may further interact and pair to form an interface, via covalent or non-covalent interactions, for example, via Van der Waals forces, hydrogen bonds, water-mediated hydrogen bonds, salt bridges or other electrostatic forces, attractive interactions between aromatic side chains, the formation of disulfide bonds, or other forces known to one skilled in the art.

"Percent (%) identity" as referring to nucleic acid or amino acid sequences herein is defined as the percentage of residues in a candidate sequence that are identical with the residues in a selected sequence, after aligning the sequences for optimal comparison purposes. The percent sequence identity comparing nucleic acids is determined using the AlignX application of the Vector NTI Program Advance 11.5.2 software using the default settings, which employ a modified ClustalW algorithm (Thompson, J.D., Higgins, D.G., and Gibson T.J. (1994) Nuc. Acid Res. 22: 4673-4680), the swgapdnamt score matrix, a gap opening penalty of 15 and a gap extension penalty of 6.66. Amino acid sequences are aligned with the AlignX application of the Vector NTI Program Advance 11.5.2 software using default settings, which employ a modified ClustalW algorithm (Thompson, J.D., Higgins, D.G., and Gibson T.J., 1994), the blosum62mt2 score matrix, a gap opening penalty of 10 and a gap extension penalty of 0.1.

### "Plurality" means two or more.

A "variant" of an antibody as described herein may comprise a functional part, functional derivative, derivative and/or analogue of an antibody. This includes antibody mimetics, monobodies and aptamers. A variant typically maintains the binding specificity of the antibody, for example the specificities of a bispecific antibody. A functional derivative of an antibody as described herein is a protein comprising a variable domain that binds one target and a variable domain that binds a second target that are linked by a linking region. The variable domains may be variable domains as such, or Fab fragments or variable domain like molecules such as single chain Fv (scFv) fragments comprising a VH and a VL linked together via a linker. Other examples of variable domain like molecules are so-called single domain antibody fragments. A single-domain antibody fragment (sdAb) is an antibody fragment with a single monomeric variable antibody region. Like a whole antibody, it is able to bind selectively to a specific antigen. With a molecular weight of only 12- 15 kDa, single-domain antibody fragments are much smaller than common antibodies (150-160 kDa) which are composed of two heavy protein chains and two light chains, and even smaller than Fab fragments (-50 kDa, one light chain and half a heavy chain) and single-chain variable fragments (-25 kDa, two variable regions, one from a light and one from a heavy chain). Single domain antibodies by themselves are not much smaller than normal antibodies (being typically 90-100kDa). Single-domain antibody fragments are mostly engineered from heavy-chain antibodies found in camelids; these are called VHH fragments (Nanobodies®). Some fishes also have heavy-chain only antibodies (IgNAR, 'immunoglobulin new antigen receptor'), from which single-domain antibody fragments called VNAR fragments can be obtained. An alternative approach is to split the dimeric variable domains from common immunoglobulin G (IgG) from humans or mice into monomers. Although most research into single-domain antibodies is currently based on heavy chain variable domains, nanobodies derived from light chains have also been shown to bind specifically to target epitopes. Other non-limiting examples of variable domain-like molecules are VHH, Human Domain Antibodies (dAbs) and Unibodies. Preferred functional parts are parts that comprise variable domains comprising a heavy chain variable region and a light chain variable region. Non-limiting examples of such variable domains are F(ab)-fragments and Single chain Fv fragments. Bispecific formats for variable domain(-like) linkage are for instance Human Serum Albumin (HSA) bound to two different scFv; bispecific mini-antibodies comprising two different scFv bound together via dimerization motifs or self-associating secondary structures such as helix bundles or coiled coils to bring about dimerization of the scFv fragments (Morrison (2007) Nat. Biotechnol. 25:1233-34). Examples of suitable HSA linkers and method for coupling scFv to the linker are described in WO2009/126920.

A functional derivative can be an antibody mimetic, a polypeptide, an aptamer or a combination thereof. These proteins or aptamers typically bind to one target. The protein of the invention binds to two or more targets. It is to be understood that any combination of these antibodies, antibody mimetics, polypeptides and aptamers can be linked together by methods known in the art. For example, in some embodiments the binding molecule of the invention is a conjugate or a fusion protein. An antibody mimetic is a polypeptide that, like antibodies, can specifically bind an antigen, but that is not structurally related to antibodies. Antibody mimetics are usually artificial peptides or proteins with a molar mass of about 3 to 20 kDa. Common advantages over antibodies are better solubility, tissue penetration, stability towards heat and enzymes, and comparatively low production costs. Non-limiting examples of antibody mimetics are affibody molecules (typically based on the Z domain of Protein A); affilins (typically based on Gamma-B crystalline or Ubiquitin); affimers (typically based on Cystatin); affitins (typically based on Sac7d from Sulfolobus acidocaldarius); alphabodies (typically based on Triple helix coiled coil); anticalins (typically based on Lipocalins); avimers (typically based on A domains of various membrane receptors); DARPins (typically based on ankyrin repeat motif); fynomers (typically based on SH3 domain of Fyn 7); kunitz domain peptides (typically based on Kunitz domains of various protease inhibitors); and monobodies (typically based on type III domain of fibronectin).

Monobodies are synthetic binding proteins that are constructed using a fibronectin type III domain (FN3) as a molecular scaffold. Monobodies are simple and robust alternative to antibodies for creating target-binding proteins. The term "monobody" was coined in 1998 by the Koide group who published the first paper demonstrating the monobody concept using the tenth FN3 domain of human fibronectin. Monobodies and other antibody mimetics are typically generated from combinatorial libraries in which portions of the scaffold are diversified using molecular display and directed evolution technologies such as phage display, mRNA display and yeast surface display. A large number of antibody mimetics have high affinity and high specificity to their respective targets. Aptamers are oligonucleotide or peptide molecules that bind to a specific target molecule. Aptamers are usually created by selecting them from a large random sequence pool, but natural aptamers also exist in riboswitches. Aptamers can be used for both basic research and clinical purposes as macromolecules. "Non-bonded" interactions" act between atoms which are not linked by covalent bonds. Accordingly these are bonds that do not involve the sharing of electrons, but rather involve the more dispersed variations of electromagnetic interactions between molecules or within a molecule. Non-bonded interactions include electrostatic interactions, such as hydrogen bonding, ionic interactions and Halogen bonding. Van der Waals forces are a subset of electrostatic interactions involving permanent or induced dipoles (or multipoles). These include the following: permanent dipole-dipole interactions, dipole-induced dipole interactions and induced dipole-induced dipole interactions. Salt bridges are a combination of two non-covalent interactions: hydrogen bonding and ionic bonding. Hydrophobic interactions are interaction of non-polar (un-ionizable) hydrocarbon molecules forced together because of stronger water:water interaction.

### Nucleic acids and vectors

The invention also provides nucleic acids encoding a polypeptide, antibody, antibody variant or antibody fragment of the invention.

The nucleic acids described herein may be used to produce a polypeptide, antibody, antibody variant or antibody fragment of the invention. Accordingly, vectors (e.g. expression vectors) comprising such nucleic acids are also provided, which can be used to produce a polypeptide, antibody, antibody variant or antibody fragment of the invention.

Antibodies are typically produced by cells that comprise nucleic acids encoding the polypeptides that together assemble to form an antibody. The nucleic acids employed to make the polypeptides of an antibody may be placed in any suitable expression vector and, in appropriate circumstances, two or more vectors may be placed in a single host cell. Generally, nucleic acids encoding modified VH domains may be cloned with the appropriate linkers and/or constant regions and the sequences are placed in operable linkage with a promoter in a suitable expression construct in a suitable cell line for expression.

The vector DNA into which the nucleic acid encoding the modified VH domain can be introduced, e.g. by cloning or synthetically, preferably comprises a nucleic acid encoding a signal peptide and the first, or first two, amino acids of a VH domain. As such, the vector DNA may be used as a standard vector for the production of modified antibodies or heavy chains as described herein, thereby omitting the need for having to alter the first, or first two, amino acids of each single VH domain. This vector DNA does thus not yet comprise the nucleic acid encoding the modified VH domain. A person skilled in the art knows how to incorporate the nucleic acid encoding the modified VH domain into such vector DNA, such that a functional antibody or heavy chain is produced, e.g. by omitting the codons encoding the first, or first two, amino acids of the nucleic acid encoding the modified VH domain that are already present in the vector DNA.

The vector may be any suitable vector, for example a phagemid (for expression in a phage) or a plasmid (for expression in a bacterial or eukaryotic cell).

A phagemid is a DNA based cloning vector which has both bacteriophage and plasmid properties. Phagemids carry an origin of plasmid replication and an origin of replication derived from bacteriophage. Phagemids can be used as a type of cloning vector in combination with filamentous phage M13 and can be packaged into the capsid of a bacteriophage. Phagemids are used in a variety of biotechnology applications; for example, they can be used in phage display (details of which are provided elsewhere herein). Several different phagemids are commercially available and can be used in the context of the invention.

The invention also provides a phage comprising a nucleic acid, or vector of the invention. Such phage may be part of a library, e.g. a phage display library.

Plasmids are also well known. Plasmids may be constructed for bacterial or mammalian expression of immunoglobulin heavy and light chain genes whose variable regions are produced (and modified) by polymerase chain reaction (PCR) as described elsewhere herein. Several different plasmids are commercially available and can be used in the context of the invention.

### Screening, host cells and methods of producing polypeptides of the invention

The invention also provides methods for the preparation of a polypeptide comprising the modified VH domain described elsewhere herein. VH nucleic acids encoding a VH domain may be provided by immunizing a non-human animal, preferably a transgenic non-human animal, with an antigen thereby producing VH domains specific for that antigen and resulting in clonal expansion of B cells producing such VH domains. Nucleic acids encoding the VH domains can then be isolated for cDNA synthesis and used in the methods described herein.

In one method, this cDNA can be used for the creation of phage display libraries in order to screen for VH domains that exhibit desirable binding properties. After selection, the desired VH nucleic acids can be transfected into a host cell for antibody production.

In another method, the cDNA is used in a frequency analysis wherein the cDNA encoding heavy chain variable regions are subjected to high throughput sequencing and the cDNAs are selected for transfection into a host cell for antibody production, including based on the frequency of the variable region gene segment used, the total variable region sequence, the HCDR3, or other features desirable to the skilled artisan.

Transfection into a host cell can be performed as known in the art and as further described herein. For example, any of the vectors as described herein may be used for the transfection. It is further contemplated that the host cell comprises a nucleic acid encoding a modified VH domain, comprising variants encoded preferably at the first and second amino acid(s) positions, integrated in its genome, and which may further comprise additional downstream variations.

A further method comprises: providing a cell which comprises one or more nucleic acids encoding polypeptides which are capable of assembly into an antibody of the invention; and cultivating said cell under conditions to provide for expression of the polypeptides and for their assembly into an antibody.

The nucleic acid molecules encoding the modified VH domain described herein may be present as extrachromosomal copies and/or stably integrated into the chromosome of the host cell. The latter is preferred in which case a locus may be targeted that is known for lack of gene silencing.

To obtain expression of nucleic acids encoding CH3 domain-comprising polypeptides, it is well known to those skilled in the art that sequences capable of driving such expression can be functionally linked to the nucleic acids encoding CH3 domain-comprising polypeptides. Functionally linked is meant to describe that the nucleic acids encoding CH3 domain-comprising polypeptides or precursors thereof are linked to sequences capable of driving expression such that these sequences can drive expression of the CH3 domain-comprising polypeptides or precursors thereof. Useful expression vectors are available in the art, e.g. the pcDNA vector series of Invitrogen. Where the sequence encoding the polypeptide of interest is properly integrated with reference to sequences governing the transcription and translation of the encoded polypeptide, the resulting expression cassette is useful to produce the polypeptide of interest, referred to as expression. Sequences driving expression may include promoters, enhancers and the like, and combinations thereof. These should be capable of functioning in the host cell, thereby driving expression of the nucleic acids that are functionally linked to them. Promoters can be constitutive or regulated, and can be obtained from various sources, including viruses, prokaryotic, or eukaryotic sources, or artificially designed. Expression of nucleic acids of interest may be from the natural promoter or derivative thereof or from an entirely heterologous promoter. Some well-known and much used promoters for expression in eukaryotic cells comprise promoters derived from viruses, such as adenovirus, e.g. the E1A promoter, promoters derived from cytomegalovirus (CMV), such as the CMV immediate early (IE) promoter, promoters derived from Simian Virus 40 (SV40), and the like. Suitable promoters can also be derived from eukaryotic cells, such as methallothionein (MT) promoters, elongation factor 1α (EF-1α) promoter, actin promoter, an immunoglobulin promoter, heat shock promoters, and the like. Any promoter or enhancer/promoter capable of driving expression of the sequence of interest in the host cell is suitable in the invention. In one embodiment the sequence capable of driving expression comprises a region from a CMV promoter, preferably the region comprising nucleotides -735 to +95 of the CMV immediate early gene enhancer/promoter. The skilled artisan will be aware that the expression sequences used in the invention may suitably be combined with elements that can stabilize or enhance expression, such as insulators, matrix attachment regions, STAR elements (WO 03/004704), and the like. This may enhance the stability and/or levels of expression.

Protein production in recombinant host cells has been extensively described, e.g. in Current Protocols in Protein Science, 1995, Coligan JE, Dunn BM, Ploegh HL, Speicher DW, Wingfield PT, ISBN 0-471-11184-8; Bendig, 1988. Culturing a cell is done to enable it to metabolize, and/or grow and/or divide and/or produce recombinant proteins of interest. This can be accomplished by methods well known to persons skilled in the art, and includes but is not limited to providing nutrients for the cell. The methods comprise growth adhering to surfaces, growth in suspension, or combinations thereof. Several culturing conditions can be optimized by methods well known in the art to optimize protein production yields. Culturing can be done for instance in dishes, roller bottles or in bioreactors, using batch, fedbatch, continuous systems, hollow fiber, and the like. In order to achieve large scale (continuous) production of recombinant proteins through cell culture, cells capable of growing in suspension are employed, and the cells are capable of being cultured in the absence of animal- or human-derived serum or animal- or human-derived serum components. Thus purification is easier and safety is enhanced due to the absence of additional animal or human proteins derived from the culture medium, while the system is also very reliable as synthetic media are the best in reproducibility.

Immunoglobulin-like polypeptides are expressed in host cells and are harvested from the cells or, preferably, from the cell culture medium by methods that are generally known to the person skilled in the art. After harvesting, these Ig-like polypeptides may be purified by using methods known in the art. Such methods may include precipitation, centrifugation, filtration, size-exclusion chromatography, affinity chromatography, cation- and/or anion exchange chromatography, hydrophobic interaction chromatography, and the like. For a mixture of antibodies comprising IgG polypeptides, protein A or protein G affinity chromatography can be suitably used (see e.g. US patents 4,801,687 and 5,151,504).

Following capture using affinity chromatography, orthogonal polishing steps are used to remove any remaining process-related and/or product related impurities, which may include homodimers, charge variants, host cell protein (HCP), and host cell DNA. In general, to obtain a purified bispecific antibody or multivalent multimer, the following steps are undertake, including host cell culture, harvest clarification, followed by protein capture, anion exchange chromatography, including to remove host cell DNA, then cation exchange chromatography (CIEX) is used to remove host cell protein, leached protein A,potential aggregates and potential product related impurities, followed by additional steps, such as nanofiltration as a final virus removal process step. Persons of skill in the art are aware the order of such steps may be modified or individual steps substituted. For example, alternatives for the second polishing step include hydrophobic interaction chromatography and mixed-mode chromatography.

Immunoglobulin-like polypeptides, and/or mixtures thereof, produced with methods according to the present invention preferably have a common light chain. Further provided is, therefore, a method according to the invention, further comprising providing said host cell with a nucleic acid encoding a common light chain. This is a light chain that is capable of pairing with at least two different heavy chains, thereby forming functional antigen binding domains. A functional antigen binding domain is capable of specifically binding to an antigen. In one embodiment, a common light chain is used that is capable of pairing with all heavy chains produced with a method according to the invention, thereby forming functional antigen binding domains, so that mispairing of unmatched heavy and light chains is avoided. In one aspect, only common light chains with one identical amino acid sequence are used. Alternatively, those of skill in the art will recognize that "common" also refers to functional equivalents of the light chain of which the amino acid sequence is not identical. Many variants of said light chain exist wherein mutations (deletions, substitutions, additions) are present that do not materially influence the formation of functional binding regions. Such variants are thus also capable of binding different heavy chains and forming functional antigen binding domains. The term 'common light chain' as used herein thus refers to light chains which may be identical or have some amino acid sequence differences while retaining the binding specificity of the resulting antibody after pairing with a heavy chain. It is for instance possible to prepare or find light chains that are not identical but still functionally equivalent, e.g. by introducing and testing conservative amino acid changes, and/or changes of amino acids in regions that do not or only partly contribute to binding specificity when paired with the heavy chain, and the like. A combination of a certain common light chain and such functionally equivalent variants is encompassed within the term "common light chain". Reference is made to WO 2004/009618 for a detailed description of the use of common light chains. Preferably, a common light chain is used in the present invention which is a germline-like light chain, more preferably a germline light chain, preferably a rearranged germline human kappa light chain, most preferably either the rearranged germline human kappa light chain IgVκ1-39/Jκ or IGVκ3-20/Jκ.

Alternatively, the skilled person may select, as an alternative to using a common chain and to avoid mispairing of unmatched heavy and light chains, means for forced pairing of the heavy and light chain, through means that are known to persons of ordinary skill in the art.

Host cells that express the polypeptides of the invention are also provided herein. A "host cell" according to the invention may be any host cell capable of expressing recombinant DNA molecules, including bacteria such as for instance Escherichia (e.g. E. coli), Enterobacter, Salmonella, Bacillus, Pseudomonas, Streptomyces, yeasts such as S. cerevisiae, K. lactis, P. pastoris, Candida, or Yarrowia, filamentous fungi such as Neurospora, Aspergillus oryzae, Aspergillus nidulans and Aspergillus niger, insect cells such as Spodoptera frugiperda SF-9 or SF-21 cells, and preferably mammalian cells such as Chinese hamster ovary (CHO) cells, BHK cells, mouse cells including SP2/0 cells and NS-0 myeloma cells, primate cells such as COS and Vero cells, MDCK cells, BRL 3A cells, hybridomas, tumor-cells, immortalized primary cells, human cells such as W138, HepG2, HeLa, HEK293, HT1080 or embryonic retina cells such as PER.C6, and the like. Often, the expression system of choice will involve a mammalian cell expression vector and host so that the antibodies can be appropriately glycosylated. A human cell line can be used to obtain antibodies with a completely human glycosylation pattern. The conditions for growing or multiplying cells (see e. g. Tissue Culture, Academic Press, Kruse and Paterson, editors (1973)) and the conditions for expression of the recombinant product may differ somewhat, and optimization of the process is usually performed to increase the product proportions and/or growth of the cells with respect to each other, according to methods generally known to the person skilled in the art. In general, principles, protocols, and practical techniques for maximizing the productivity of mammalian cell cultures can be found in Mammalian Cell Biotechnology: a Practical Approach (M. Butler, ed., IRL Press, 1991). Expression of antibodies in recombinant host cells has been extensively described in the art. The nucleic acids encoding the light and heavy chains may be present as extrachromosomal copies and/or stably integrated into the chromosome of the host cell.

### Libraries

The invention provides libraries (i.e. collections) of distinct nucleic acids, vectors or phages of the invention. The libraries may comprise at least about 10⁶ distinct nucleic acids, vectors or phages of the invention.

An example of a library according to the invention is a display library. Methods of preparing a display library are well known in the art. For example, a method for the preparation of a display library displaying a variety of modified VH domains of the invention, may comprise integrating a nucleic acid of the invention (e.g. in the form of a vector described elsewhere herein) into an organism, such as a phage or yeast, or other vessel for peptide display, wherein said organism expresses and displays said modified VH domain on the surface of said organism or vessel. Multiple modified VH domains, typically multiple different modified VH domains may be displayed on the surface of multiple organisms, such as phages (each phage displaying one modified VH domain) by use of phage display library. Thus, in a display library, a plurality of modified VH domains encoded by nucleic acids of the invention may be paired with a human common chain variable region. The display library may be, for example, a Fab phage display library.

### Display library Technology

Various forms of display technologies including phage display, yeast display, ribosome display, mRNA display, among others, are known in the art, and encompassed by the invention described herein, for use with the modified VH domains described herein. The following discussion focuses on phage display, but such description is not limiting and based on the description provided herein, could readily be applied to other forms of display technology. Phage display is a prominent technique used including for the study of protein-protein, protein-peptide, and protein-DNA interactions that uses bacteriophages which are viruses that infect bacteria. Many of the protocols described herein are standard protocols for the construction of phage display libraries and the panning of phages for binding to an antigen of interest and are described in Antibody Phage Display: Methods and Protocols (editor(s): Philippa M. O'Brien, Robert Aitken). Libraries may be grown and harvested according to procedures know in the art, for example, as described by Kramer et al. 2003 (Kramer et al. 2003. Nucleic Acids Res.31(11): e59) using VCSM13 (Stratagene) as helper phage strain. Phages may be grown and processed according to procedures known in the art, for example, as described by Kramer et al. 2003 (Kramer et al. 2003. Nucleic Acids Res. 31(11): e59) using VCSM13 as helper phage strain.

In the exemplary technique, a nucleic acid encoding a protein of interest, for example a nucleic acid encoding a modified VH domain, is integrated into a phage coat protein gene, causing the phage to "display" the protein on its outside while containing the nucleic acid encoding for the protein on its inside. In this way, a connection between genotype and phenotype is established. With regard to antibody discovery, in phage display, large collections (libraries) of VH and/or VL domains may be expressed on the surface of filamentous bacteriophage particles so that they pair to form binding domains. From these libraries, phages may be selected through binding interaction with an antigen and the displayed binding domain. Thus, the displaying phages can be screened against other proteins, peptides or DNA sequences, or other forms of targets moieties, to detect interaction between the displayed VH, VL or binding domain and those other moieties. In this way, large libraries of VH, VL or binding domains can be screened and amplified in a process called in vitro selection, which is analogous to natural selection. Accordingly, a modified VH domain of the invention may be displayed on phage.

The invention described herein provides an efficient assembly-line process of obtaining essentially all of the nucleic acids encoding heavy chain variable regions from an immunized animal, including a transgenic animal, and integrating nucleic acids encoding modified heavy chain variable regions into a display technology (e.g., phage, yeast, ribosomal etc.), wherein each of said nucleic acids encodes for a non-glutamate and non-glutamine amino acid residue at the N-terminus of the modified heavy chain variable region, thereby permitting testing of essentially all the heavy chain variable regions from the immunize animal lacking such residue, irrespective of the human variable region gene segment or from which the variable region is derived in said animal.

Alternatively, the invention provides for a method of producing a defined population of binding molecules, comprising N-terminal modified heavy chain variable regions, whereby a population of B cells expressing a limited VL repertoire, preferably a single or common light chain and expressing a variety of heavy chain variable regions specific to an antigen of interest, are obtained. Said B cells may be obtained after immunization of a transgenic animal harbouring a human immunoglobulin locus or loci with the antigen of interest. Nucleic acids (RNA or DNA) from said B cells are sequenced encoding a portion and preferably essentially all of said heavy chain variable regions. Said nucleic acids encoding the immunoglobulin heavy chain variable regions in said sample are preferably amplified, and subjected to a frequency analysis, wherein the V gene segment usage with said population are analysed, the VH sequence is analysed, the HCDR3, and additional qualities of the repertoire of interest to the skilled artisan.

Said heavy chain variable region(s) from this frequency analysis is then selected and provided into a host cell in a manner described herein to produce variations comprising the first or first and second encoded amino acids of the variable region of at least one VH sequence, preferable two or more with at least one VL sequence of said limited VL repertoire or common light chain further provided into said host cell. Thereafter, said host cell is cultured to allow expression of the modified VH and VL polypeptides, wherein one modified VH is provided with one VL into said host cell to produce a monospecific antibody, and wherein two or more modified VH sequences are provided with one VL into said host cell to produce a multispecific antibody.

### Methods of simultaneously amplifying and modifying a nucleic acid

A method of simultaneously amplifying and modifying a nucleic acid that encodes a human immunoglobulin heavy chain variable domain is also provided herein. The method is particularly useful as it combines amplification of a target template with modification of the template sequence in a single step such that the amplified nucleic acid encodes a novel polypeptide of the invention (i.e. a polypeptide comprising a modified human VH domain as described elsewhere herein).

The method described herein comprises the step of providing a nucleic acid that encodes a human immunoglobulin heavy chain variable domain (also referred to as a human VH domain herein). The nucleic acid that is provided for use in the method may also be referred to herein as the template sequence, which is a sequence that is to be amplified and modified.

The template nucleic acid may be obtained from any suitable source. Typically, the template nucleic acid may be cDNA, for example cDNA that has been generated by reverse transcription of an RNA sample. The RNA sample may be total RNA or mRNA obtained from a cell that expresses a polypeptide comprising a human immunoglobulin heavy chain variable domain.

Any host cell described herein may be used to obtain the template nucleic acid (e.g. a cDNA sequence that corresponds to an RNA sequence produced by the cell that encodes a human immunoglobulin heavy chain variable domain). In a particularly advantageous example, the template nucleic acid is obtained from human cells or transgenic animal cells comprising human immunoglobulin variable region gene segments. In another example, the transgenic animal comprises a human immunoglobulin heavy chain locus, or portion thereof (e.g. a human immunoglobulin heavy chain mini-locus).

Any suitable transgenic animal may be used, for example a transgenic sheep, rabbit, rat, mouse, bird, including chicken, etc. comprising human variable region gene segments, which form human, humanized or chimeric antibodies or heavy chains comprising at the N-terminus of the heavy chain variable domain a glutamate or glutamine.

Transgenic animals harbouring human variable region gene segments have been described previously. Such transgenic animals may be used in the methods described herein. A transgenic animal suitable for use in an invention described herein harbour nucleic acids encoding for a human common immunoglobulin chain comprising a rearranged light or heavy variable chain and encoding an unrearranged variable region of the cognate chain(s) in the germ line of such animals as described in WO2009/157771. Such transgenic animals are capable of producing antibodies having diversity generated through one of the two cognate chains of the immunoglobulin, e.g., the unrearranged heavy or light chain, which undergoes somatic recombination during B-cell development and affinity maturation after antigen exposure. These transgenic animals, such as MeMo®, are capable of producing diverse repertoires of antibodies against an array of antigens.

The human transgenic animal may be immunised with an antigen or epitope of interest. A suitable immunization protocol is typically one that causes the selective expansion of B cells, meaning that primary and booster immunizations are designed to cause selective expansions of B cells that produce antibodies that bind to the antigen or epitope of interest. The immunization protocol may for example use different forms or fragments of the antigen during primary immunization and each subsequent booster immunization. For example, the antigen may be expressed on the membrane of a cell, a lipoparticle, a micelle, a recombinant protein, a recombinant protein fused to another protein, a domain of a protein or a peptide of a protein. The immunization protocol may include the use of an adjuvant during the primary and/or booster immunizations. An adjuvant may be used during primary immunization only to limit the extent of nonspecific expansion of bystander B cells. Bystander B cells are cells that are activated without the step of binding of antigen to the antibody receptor expressed on the surface of the B cell. It is known in the art that immunization with Fc-fusion proteins for example, often results in a robust anti-Fc response where up to about 70% of all B cells react to the Fc part of the fusion protein rather than to the antigen of interest. An immunization protocol may be used without adjuvant to preferentially expand B cells that have been activated by the antigen used for immunization.

When obtaining a nucleic acid encoding a human immunoglobulin heavy chain variable domain from a transgenic animal, B cells are typically recovered. B cells may be recovered from any suitable source, such as a tissue (for example from lymph tissue or from bone marrow (i.e. from tissue producing B cells)) or from peripheral blood (e.g. for larger transgenic animals such as sheep). For example, magnetic micro-beads coated with the human pan B-cell marker, CD19 may be used to isolate B cells from peripheral blood (see e.g. Bertrand, F. E., III, et al., Blood 90 (1997) 736-744).

Nucleic acids encoding a human immunoglobulin heavy chain variable domain (e.g. RNA sequences) are typically isolated from the cells using standard techniques. Reverse transcription using gene specific primers or generic RNA primers (e.g. polyA primers) may be used to obtain corresponding cDNA. Typically, this cDNA, which also encodes a human immunoglobulin heavy chain variable domain, is used as the template nucleic acid in the methods described herein.

The method also comprises the step of performing a polymerase chain reaction (PCR) with at least one 5' primer, at least one 3' primer and the nucleic acid to generate an amplified nucleic acid.

The terms "polymerase chain reaction" and "PCR", are used interchangeably herein. They refer to a method for specifically amplifying a region of nucleic acids, e.g. of DNA or RNA. The region can be a single gene, a part of a gene, a coding or a non-coding sequence or comprise a combination of these. Most PCR methods typically amplify DNA fragments of hundreds of base pairs (bp), although some techniques allow for amplification of fragments up to 40 kilo base pairs (kb) in size. A basic PCR set up requires several components and reagents. These components include a nucleic acid template that contains the region to be amplified, two primer complementary to the 5'- and 3'-end of the region to be amplified ("5' primer" (or forward primer) and "3' primer" (or reverse primer) respectively), a polymerase, such as Taq polymerase or another thermostable polymerase, deoxynucleotide triphosphates (dNTPs) from which the polymerase synthesizes a new strand, a buffer solution providing a suitable chemical environment for optimum activity and stability of the polymerase, divalent cations, generally Mg²⁺, and finally, monovalent cations like potassium ions.

The exact PCR conditions required for amplifying the template nucleic acid can be determined by a person of ordinary skill in the art, using their common general knowledge. Non-limiting examples of PCR conditions that may be used in the context of the invention include those described elsewhere herein.

The method uses at least one 5' primer that comprises a nucleic acid with a modification site that introduces a modification in the amplified nucleic acid such that the amplified nucleic acid encodes a human immunoglobulin heavy chain variable domain comprising an N-terminal amino acid that is not glutamine or glutamate. The residue may be selected from the group consisting of: alanine, arginine, asparagine, aspartate, glycine, histidine, isoleucine, leucine, lysine, methionine, phenylalanine, proline, serine, threonine, tryptophan, tyrosine and valine. This allows the method to amplify the template nucleic acid whilst simultaneously modifying it such that it encodes a polypeptide of the invention (i.e. a polypeptide comprising a modified human VH domain as described herein).

The at least one 5' primer may have any sequence that introduces the required modification in the amplified nucleic acid. Several examples of suitable 5' primers are provided herein. Other primer sequences that are also capable of introducing the required modification at the required position(s) in the encoded VH domain can also be used. Once the skilled person is aware that, surprisingly, modifications at the N-terminus of the human VH domain can be tolerated, design of suitable primers is possible.

The at least one 5' primer used in the methods of the invention may include a sequence that introduces a modification in the encoded VH domain of the template nucleic acid such that in the amplified nucleic acid the N-terminal residue glutamine or glutamate of the encoded human VH domain is replaced with an acidic or polar residue, such as aspartate or asparagine. These amino acids have similar biochemical properties to glutamine or glutamate and thus may be useful choices.

In a particular example, the at least one 5' primer used in the methods of the invention may include a sequence that introduces a modification in the encoded VH domain of the template nucleic acid such that in the amplified nucleic acid the N-terminal residue glutamine or glutamate of the encoded human VH domain is replaced with alanine. This modification is particularly useful as it eliminates pyroglutamate formation and also maintains (e.g. improves) signal peptide cleavage efficiency.

Alanine is an aliphatic residue. Accordingly, in another example, the at least one 5' primer used in the methods of the invention may include a sequence that introduces a modification in the encoded VH domain of the template nucleic acid such that in the amplified nucleic acid the N-terminal residue glutamine or glutamate of the encoded human VH domain is replaced an aliphatic residue such as alanine, glycine, alanine, valine, leucine or isoleucine.

The at least one 5' primer used in the methods of the invention may include a sequence that introduces two modifications in the encoded VH domain of the template nucleic acid such that in the amplified nucleic acid the N-terminal sequence of the encoded human VH domain is replaced with a (first) N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine; and a (second) amino acid (as calculated from the N-terminus of the VH domain) selected from the group consisting of proline, valine, aspartate, glutamate, serine, leucine or threonine. In one specific example, the second amino acid at the N-terminus of the VH domain is selected to be proline.

In one particular example, the at least one 5' primer used in the methods of the invention may include a sequence that introduces two modifications in the encoded VH domain of the template nucleic acid such that in the amplified nucleic acid the N-terminal sequence of the encoded human VH domain is replaced with alanine-proline, alanine-aspartate, alanine-glutamate, alanine-threonine, alanine-valine, alanine-serine and alanine-leucine. As used herein the formatting used for "alanine-proline" etc refers to the two adjacent amino acids at the N-terminal end of the modified VH domain (i.e. the "first-second" amino acids at the N-terminus of the VH domain (in a N-terminal to C-terminal direction)).

In a preferred example, the at least one 5' primer used in the methods of the invention includes a sequence that introduces two modifications in the encoded human VH domain of the template nucleic acid such that in the amplified nucleic acid the first two amino acids at the N-terminus of the encoded human VH domain are alanine-proline. This combination was found to be particularly advantageous for promoting signal peptide cleavage efficiency.

The at least one 5' primer used in the methods of the invention may include a sequence that encodes a signal peptide or portion of a signal peptide such that in the amplified (and modified) nucleic acid, a signal peptide is encoded upstream of the encoded modified human VH domain. The spatial relationship between the signal peptide and the N-terminus of the modified VH domain in the encoded polypeptide is described in detail elsewhere herein and applies equally here.

The at least one 5' primer used in the methods of the invention may therefore include a sequence that encodes a signal peptide or portion of a signal peptide comprising the sequence .....AQPAMA (SEQ ID NO: 5) upstream of the modification site (i.e. such that in the encoded polypeptide, the sequence ... AQPAMA (SEQ ID NO: 5) of the signal peptide is directly adjacent to (and upstream of) the modified N-terminus of the modified VH domain). For example, the at least one 5' primer may include a sequence that introduces an alanine (or an alanine-proline combination) at the N-terminus of the modified VH domain encoded by the amplified nucleic acid and introduces a signal peptide upstream of the encoded modified VH domain such that the residues flanking the signal peptide cleavage site in the modified VH domain comprise **AQPAMA**A (SEQ ID NO: 6) or **AQPAMA**AP (SEQ ID NO: 7) (with the sequence at the C-terminal end of the signal peptide in bold, and the N-terminal amino acid of the VH domain underlined).

In a further example, the at least one 5' primer used in the methods of the invention may include a sequence that encodes a signal peptide or portion of a signal peptide comprising the sequence .....AQPAMA (SEQ ID NO: 5) upstream of the modification site (i.e. such that in the encoded polypeptide, the sequence ... AQPAMA (SEQ ID NO: 5) of the signal peptide is directly adjacent to (and upstream of) the modified N-terminus of the modified VH domain). For example, the at least one 5' primer may include a sequence that introduces an alanine (or an alanine-proline combination) at the N-terminus of the modified VH domain encoded by the amplified nucleic acid and introduces a signal peptide upstream of the encoded modified VH domain such that the residues flanking the signal peptide cleavage site in the modified human VH domain comprise MKYLLPTAAAGLLLLA**AQPAMA**A (SEQ ID NO: 8) or MKYLLPTAAAGLLLLA**AQPAMA**AP (SEQ ID NO: 9) (with the sequence at the C-terminal end of the signal peptide in bold, and the N-terminal amino acid of the VH domain underlined).

In a further example, the at least one 5' primer used in the methods of the invention may include a sequence that encodes a signal peptide or portion of a signal peptide comprising the sequence .....AQPAMA (SEQ ID NO: 5) upstream of the modification site (i.e. such that in the encoded polypeptide, the sequence ... AQPAMA (SEQ ID NO: 5) of the signal peptide is directly adjacent to (and upstream of) the modified N-terminus of the modified VH domain). For example, the at least one 5' primer may include a sequence that introduces an alanine (or an alanine-proline combination) at the N-terminus of the modified VH domain encoded by the amplified nucleic acid and introduces a signal peptide upstream of the encoded modified VH domain such that the residues flanking the signal peptide cleavage site in the modified human VH domain comprise MGWSCIILFLVLLL**AQPAMA**A (SEQ ID NO: 10) or MGWSCIILFLVLLL**AQPAMA**AP (SEQ ID NO: 11) (with the sequence at the C-terminal end of the signal peptide in bold, and the N-terminal amino acid of the VH domain underlined).

Examples of suitable 5' primer sequences that modify the first two codons of the N-terminus of the human VH domain (to encode alanine-proline) and introduce a signal peptide directly adjacent and upstream of the encoded modified VH domain are provided in the table below. As will be seen from the table, the exemplified 5' primers are designed for amplification of each functional human IGHV gene segment from each gene family. This is based on the sequences after the modification site in the primer, which are complementary (at least in part) to the unmodified nucleic acid found at these positions in the listed human VH gene segments.

**Table 1: list of 5' universal primers and their target human IGHV family.**

| **Human IGHV family:** | **5' universal primers described herein that are suitable for amplification and AP modification of the IGHV family**: |
|---|---|
| IGHV1 | 1308AP, 1308AP2, 2018AP2, 2018AP or 2020AP2 |
| IGHV2 | 1310AP2, 1310AP3, 1310AP4 or preferably 1310AP5 |
| IGHV3 | 0508AP, 0508AP2, 2018AP, 2018AP2, 2021AP, 2021AP2, 2021AP3, 2021AP4 or 2021AP5 |
| IGHV4 | 1312AP2/2019AP2 |
| IGHV5 | 1313AP or 1313AP2 |
| IGHV6 | 1310AP2, 1310AP3 and 1310AP4, 1310AP5, 1312AP2/2019AP2, |
| IGHV7 | 1314AP or 1314AP2 |

The method described herein may therefore comprise:
(a) amplifying and modifying a nucleic acid encoded by an IGHV1 family gene using a 5' primer that is selected from 1308AP, 1308AP2, 2018AP, 2018AP2 or 2020AP2; and/or
(b) amplifying and modifying a nucleic acid encoded by an IGHV2 family gene using a 5' primer that is selected from 1310AP2, 1310AP3, 1310AP4, or 1310AP5; and/or
(c) amplifying and modifying a nucleic acid encoded by an IGHV3 family gene using a 5' primer that is selected from 0508AP, 0508AP2, 2018AP, 2018AP2, 2021AP2, 2021AP, 2021AP3, 2021AP4, or 2021AP5; and/or
(d) amplifying and modifying a nucleic acid encoded by an IGHV4 family gene using a 5' primer that is selected from 1312AP2; and/or
(e) amplifying and modifying a nucleic acid encoded by an IGHV5 family gene using a 5' primer that is selected from 1313AP or1313AP2;
(f) amplifying and modifying a nucleic acid encoded by an IGHV6 family gene using a 5' primer that is selected from 1310AP2, 1310AP3, 1310AP4, 1310AP5 or 1312AP2; and/or
(g) amplifying and modifying a nucleic acid encoded by an IGHV7 family gene using a 5' primer that is selected from 1314AP or 1314AP2.

It is noted herein that primer 1312AP2 and 2019AP2 have an identical sequence, therefore these terms can be used interchangeably. This is also indicated herein by the use of the term "1312AP2/2019AP2".

As shown in the examples section below, certain 5' primers disclosed herein may be used preferentially.

For example, when amplifying and modifying a nucleic acid encoded by a gene segment from the IGHV1 gene family, a 5' primer that is selected from 1308AP, 1308AP2, 2018AP, 2018AP2 or 2020AP2 may be used. Alternatively a primer that similarly modifies the first two N-terminal amino acids of a human variable region to encode AP may be used. One particular example includes a 5' primer that is selected from 1308AP2, 2018AP2 or 2020AP2.

Furthermore, when amplifying and modifying a nucleic acid encoded by a gene segment from IGHV2 gene family, a 5' primer that is selected from1310AP2, 1310AP3, 1310AP4 or 1310AP5 may be used, or alternatively a primer that similarly modifies the first two N-terminal amino acids of a human variable region to encode AP may be used. One particular example includes a 5' primer that is 1310AP5.

Furthermore, when amplifying and modifying a nucleic acid encoded by a gene segment from IGHV3 gene family, a 5' primer that is selected from 0508AP, 0508AP2, 2018AP, 2018AP2, 2021AP, 2021AP2, 2021AP3, 2021AP4 or 2021AP5, may be used, or alternatively a primer that similarly modifies the first two N-terminal amino acids of a human variable region to encode AP may be used. One particular example includes a 5' primer that is selected from 0508AP, 2021AP2 or 2018AP2.

Furthermore, when amplifying and modifying a nucleic acid encoded by a gene segment from IGHV4 gene family, a 5' primer that is 1312AP2 may be used, or alternatively a primer that similarly modifies the first two N-terminal amino acids of a human variable region to encode AP may be used.

Furthermore, when amplifying and modifying a nucleic acid encoded by a gene segment from IGHV5 gene family, a 5' primer that is 1313AP or 1313AP2 may be used, or alternatively a primer that similarly modifies the first two N-terminal amino acids of a human variable region to encode AP may be used. One particular example includes a 5' primer that is 1313AP2.

Furthermore, when amplifying and modifying a nucleic acid encoded by a gene segment from IGHV6 gene family, a 5' primer that is 1310AP2, 1310AP3, 1310AP4, 1310AP5 or 1312AP2 may be used, or alternatively a primer that similarly modifies the first two N-terminal amino acids of a human variable region to encode AP may be used. One particular example includes a 5' primer that is selected from 1312AP2 and 1310AP5.

When amplifying and modifying a nucleic acid encoded by a gene segment from IGHV7 family gene, a 5' primer that is 1314AP or 1314AP2 may be used, or alternatively a primer that similarly modifies the first two N-terminal amino acids of a human variable region to encode AP may be used. One particular example includes a 5' primer that is 1314AP2.

As would be clear to a person of ordinary skill in the art, there may be circumstances in which it would be beneficial to simultaneously amplify and modify a plurality of distinct template nucleic acid that encode different human VH domains. For example, it may be beneficial to use the methods described herein to amplify (and modify) a partial or complete repertoire of different human VH domains encoded within a human and/or a human transgenic animal cell sample.

As an example, the methods described herein may be used to simultaneously modify and amplify the repertoire of nucleic acid encoding human VH domains in a transgenic animal (for example, a transgenic murine or avian organism having a human immunoglobulin heavy chain locus or portion thereof; such as a MeMo® mouse). The methods provided herein enable simultaneous amplification and modification of different IGHV gene segments, for example, using a multiplex PCR reaction in which several distinct 5' primers are used.

The terms "multiplex polymerase chain reaction" or "multiplex PCR", are used interchangeably herein to refer to a polymerase chain reaction employing multiple, unique primers in a single PCR reaction/mixture to produce amplified nucleic acids with different sequences. By targeting multiple genes at once, additional information can be obtained from a single test run that otherwise would require several times the reagents and more time to perform. Annealing temperatures for each primer sets must be optimized to work correctly within a single reaction.

The method may therefore include the step of providing a plurality of distinct nucleic acids encoded by at least one human gene segment selected from each of the following human gene families: IGHV1, IGHV2, IGHV3, IGHV4, IGHV5, IGHV6 and IGHV7. Advantageously, the plurality of distinct nucleic acids provided (as template nucleic acid) can be amplified and modified at the same time, in one PCR reaction.

For example, at least one primer from each of the rows in Table 1 may be selected and used in the methods of the invention to amplify and modify template nucleic acid that encode functional gene segments within the following human gene families IGHV1, IGHV2, IGHV3, IGHV4, IGHV5, IGHV6 and IGHV7 simultaneously.

As shown in the examples section below, certain 5' primers disclosed herein may be used preferentially.

For example, a combination of at least one primer from each of the following categories may be selected to be used in the methods of the invention (resulting in a mixture of at least six distinct 5' primers being used in one reaction, depending on the number of V gene families, which give rise to the VH repertoire):
a) a 5' primer that is selected from 1308AP, 1308AP2, 2020AP2, 2018AP, or 2018AP2;
b) a 5' primer that is selected from 1310AP2, 1310AP3, 1310AP4, or 1310AP5;
c) a 5' primer that is selected from 0508AP, 0508AP2, 2018AP, 2018AP2, 2021AP, 2021AP2, 2021AP3, 2021AP4, or 2021AP5;
d) a 5' primer that is 1312AP2;
e) a 5' primer that is selected from 1313AP, or 1313AP2;
f) a 5' primer that is selected from 1310AP2, 1310AP3, 1310AP4, 1310AP5 or 1312AP2; and
g) a 5' primer that is selected from 1314AP, or 1314AP2.

As would be clear to a person of ordinary skill in the art, a combination of at least one primer from each of a) to g) above would be particularly advantageous as it would provide a universal 5' primer mix that would simultaneously amplify and modify template nucleic acid that encode human IGHV1, IGHV2, IGHV3, IGHV4, IGHV5, IGHV6 and IGHV7 gene segments in one reaction.

There may also be circumstances where a subset of the primers listed in Table 1 (or listed in a) to g) above) may be preferred. For example, when focusing on template nucleic acid that encode human IGHV1, IGHV2, IGHV3 only, it may be useful to select a combination of primers in the corresponding rows of Table 1 (or in (a) to (c) above) only. Preference for which primers to employ correlate to, for example, the gene families within the platform used for antibody or heavy chain generation. For example, where a transgenic host harbouring a human mini-locus is used that comprises VH gene segments from gene families IGHV1, IGHV5 and IGHV7, primers listed in Table 1 corresponding to said gene families are preferably used to generate a panel of heavy chains comprising variants at the N-terminus. In addition to the primers provided above, based on the teachings described herein, a skilled artisan could further develop primers that apply across each VH gene family for amplification of any human VH domain, which is likewise included in the present invention.

The methods described herein comprise performing a polymerase chain reaction (PCR) with at least one 5' primer, at least one 3' primer and the nucleic acid to generate an amplified nucleic acid. The at least one 5' primer and the (template) nucleic acid are discussed in detail above.

Any appropriate 3' primer or a mixture of 3'primers may be used. As would be clear to a person of ordinary skill in the art, this includes 3' primers that are complementary to the nucleic acid encoding the FR4 region of the encoded human VH domain, or 3' primers that are complementary to the nucleic acid encoding the human heavy chain constant domain. For human VH domains, the FR4 region is encoded by rearranged human J gene segments (or J gene segments in the context of a common heavy chain). Accordingly, design of an appropriate 3' primer is well-known to persons of ordinary skill.

Exemplary primers are provided below, which include a region that is complementary to the end of FR4, and include restriction sites BstElI and XhoI.
HuJH1/2xho = TATTGTTACCTCGAGACGGTGACCAGGGTGCC (SEQ ID NO: 12)
HuJH3xho = TATTGTTACCTCGAGACGGTGACCATTGTCCC (SEQ ID NO: 13)
HuJH4/5xho = TATTGTTACCTCGAGACGGTGACCAGGGTTCC (SEQ ID NO: 14)
HuJH6xho = TATTGTTACCTCGAGACGGTGACCGTGGTCCC (SEQ ID NO: 15)

The methods described herein may further comprise the step of introducing each amplified and modified nucleic acid into a vector. Methods for introducing nucleic acids into vectors are well known and include restriction enzyme digestion and ligation. Suitable vectors are described elsewhere herein and include phagemids or plasmids.

The methods described herein may also further comprise transforming or transfecting each vector into a cell to generate a library. Methods for introducing vectors into cells are well known. Suitable host cells are described elsewhere herein and include phage competent cells such as phage competent *E.coli* or phage competent yeast. Corresponding libraries are also described elsewhere herein.

### Kits

Kits are also provided herein. The kits comprise a plurality of 5' primers described herein. The kit may comprise at least two, at least three, at least four, at least five, at least six, at least seven, at least eight, at least nine etc. distinct 5' primers described herein. Optionally the kits also comprise at least one 3' primer described herein, depending on the nature of the repertoire of human VH domains. Details of suitable primers is given above and applies equally here.

The components of the kit may be housed in a container that is suitable for transportation.

In addition, the kits may include instructional materials containing directions (i.e., protocols) for the use of the materials provided in the kit. While the instructional materials typically comprise written or printed materials, they may be provided in any medium capable of storing such instructions and communicating them to an end user. Suitable media include, but are not limited to, electronic storage media (e.g., magnetic discs, tapes, cartridges, chips) and optical media (e.g., CD ROM). The media may include addresses to internet sites that provide the instructional materials. Such instructions may be in accordance with any of the methods or uses detailed herein.

### Pharmaceutical compositions and methods of use

Also provided by the invention is a pharmaceutical composition which comprises an antibody, antibody fragment or an antibody variant and a pharmaceutically acceptable carrier and/or diluent. Accordingly, the invention provides an antibody, antibody fragment or an antibody variant as described herein for use in the treatment of the human or animal body by therapy. Further provided by the invention is a method for the treatment of a human or animal suffering from a medical condition, which method comprises administering to the human or animal a therapeutically effective amount of an antibody, antibody fragment or antibody variant as described herein. The amount of antibody, antibody fragment or antibody variant according to the invention to be administered to a patient is typically in the therapeutic window, meaning that a sufficient quantity is used for obtaining a therapeutic effect, while the amount does not exceed a threshold value leading to an unacceptable extent of side-effects. The lower the amount of antibody, antibody fragment or antibody variant needed for obtaining a desired therapeutic effect, the larger the therapeutic window will typically be. An antibody, antibody fragment or antibody variant according to the invention exerting sufficient therapeutic effects at low dosage is, therefore, preferred.

A reference herein to a patent document or other matter which is given as prior art is not to be taken as an admission that that document or matter was known or that the information it contains was part of the common general knowledge as at the priority date of any of the claims.

Unless defined otherwise herein, all technical and scientific terms used herein have the same meaning as commonly understood by one of ordinary skill in the art to which this invention pertains. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a", "an" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

Aspects of the invention are demonstrated by the following non-limiting examples.

### EXAMPLES

New primers have been identified that can amplify the entirety of variable regions produced from each functional VH gene segment within each human VH gene family. The new primers modify any N-terminus of any human VH domain produced by virtue of recombination of any functional human VH gene segment, resulting in prevention of N-terminal pyroglutamic acid formation and/or increased expression.

The examples below demonstrate the invention using DNA encoding variable regions produced by Merus' MeMo® mice and integrating such DNA into vectors while varying the first (or first and second) N-terminal encoded amino acids of the human immunoglobulin heavy chain variable domain. Panels of DNA encoding variable regions generated by two different MeMo® mice were successfully integrated into vectors having the first (or first and second) N-terminal encoded amino acids varied. Such MeMo® mice have synthetic heavy chain mini loci that contain a representative VH gene segment from human VH gene families. The primers are shown to work across all VH gene subfamilies. The primers have been optimised for VH amplification efficiency and VH diversity. They have been used to successfully generate phage display libraries and for subsequent Fab expression.

### Study rationale

The Merus mouse (MeMo®) lines previously described express antibodies with human VH regions. After immunization of these mice, RNA can be isolated, followed by cDNA synthesis and PCR amplification of the VH regions. Of note, VH sequences start with E or Q.

PCR primers have now been designed that replace the E or Q at the N-terminus of all VH sequences during amplification. The primers have specifically been designed to amplify unbiased VH repertoires for any heavy chain variable region comprised of any human recombined functional V gene segment and functional gene family within the human repertoire.

When changing the sequence of the N-terminus of the VH, care should also be taken not to affect antibody properties like structure, antigen binding and stability, and signal peptide (SP) cleavage. Amino acid frequencies at individual positions of SP's and of the associated mature proteins have been analyzed for 2352 secreted proteins from eukaryotes, Gram-positive bacteria and Gram-negative bacteria [Choo and Ranganathan, 2008]. This analysis showed similarities as well as differences between the species groups. Overall, amino acid preferences were mainly observed within the SP's, however certain preferences were also observed for the first few residues of the mature peptides:
From review of these data, it is noted that A and Q are preferred at the first position of both eukaryotic (25% A or Q) and Gram-negative (54% A or Q) mature proteins. In eukaryotes, P was relatively often found at the second (16%) and fourth (11%) position. In Gram-negative bacteria, D, E, P and T were frequently seen at the second position (56% of all analyzed proteins have one of these four residues). For the third and fourth position T was prevalent at the third (11%) and fourth (13%) position.

On the other hand, several amino acids are clearly under-represented at certain positions. For example, W is found at a frequency of only about 1% at the first position in eukaryotes and Gram-negative bacteria. Therefore, it is concluded that as particular amino acids are being favored or disfavored, optimization of SP cleavage may be possible by adapting the sequence of the first few residues of the mature peptides.

When changing the sequence of the VH N-terminus, the modified sequences should combine well with prokaryotic (bacterial) as well as with eukaryotic SP's.

Bacterial SPs include, for example, MKYLLPTAAAGLLLLAAQPAMA (SEQ ID NO: 1). Eukaryotic SPs include, for example, MGWSCIILFLVLLLAQPAMA (SEQ ID NO:4). These signal peptides were used as representative, non-limiting examples below.

To be able to check in silico that SP cleavage from modified VH is at least as good as for the corresponding wild-type (WT) VH, a set of eighteen representative sequences were generated that each contain a SP and the first 20 N-terminal amino acids of the VH region (9 VH regions combined with 2 different SP sequences = 18 representative sequences). The VH region sequences that were chosen were specifically selected as representative sequences for all VH gene subfamilies. VH residues beyond position 20 in the VH region are understood to not significantly affect SP cleavage [Choo and Ranganathan, 2008].

The first VH residue in each of the 18 sequences ('position 1') was varied to include all 20 possible amino acids, thereby resulting in 18 x 20 = 360 sequences. All sequences were given a code of format P#X or E#X, where:
P = prokaryotic SP; E = eukaryotic SP
# = internal designation number
X = amino acid at VH position 1

For example, P1A comprises the prokaryotic SP and the 20-residue VH sequence has the first VH encoded amino acid E changed to A.

To study the effect of the first VH residue on SP cleavage, all 360 sequences were analyzed *in silico* using prediction tool SignalP 4.1 at www.cbs.dtu.dk/services/SignalP [Petersen et al, 2011], using the following parameters:
organism group: Gram-negative bacteria' for P#X sequences and 'Eukaryotes' for E#X sequences
output format: 'Short (no graphics)'
all other parameters: standard/default

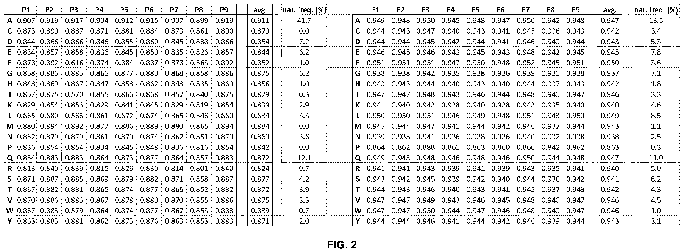

For all 360 sequences, the position of the SP cleavage site was correctly predicted. The so-called D (discrimination) scores of the prediction were compared for all 360 sequences (see **Figure 2**). A high D score indicates a high chance that the sequence preceding the VH region is in fact a signal peptide. Here it is assumed that a higher D score corresponds to a higher chance that the SP is cleaved efficiently. **Figure 2** also lists the unmodified amino acid frequencies at position 1 for a panel of 307 Gram-negative and 1877 eukaryotic proteins containing N-terminal SP's [Choo and Ranganathan, 2008; additional file 2]. Here it is assumed that a higher frequency corresponds to a higher chance that the SP is cleaved efficiently. In the P#X sequences, highest D scores are observed for X=A (average 0.911), and A is the most frequent amino acid in prokaryotic secretory proteins (41.7%). As another example, in the E#X sequences, lowest D scores are observed for X=P (average 0.863), and P is the least frequent amino acid in eukaryotic secretory proteins (0.3%).

Based on D score, the following three residues having the highest score were selected as potential alternative residues at the **first** VH position:
A: most scores/frequencies are (much) higher compared to E and Q; it is by far the most frequent amino acid in unmodified SP's
D: the side chain is chemically similar to E; the scores/frequencies are comparable to E and Q
S: part of the scores/frequencies are a bit higher compared to E and/or Q

Together with the sequences containing E or Q at position 1, this leaves 5 x 9 x 2 = 90 sequences for further analysis (5 amino acids at position 1 x 9 VH region sequences x 2 SP's).

For each of the 90 sequences (i.e. with A, D, E, Q or S at position 1), the **second** VH residue ('position 2') was varied. The following 7 residues were omitted since these are rarely or not at all found in unmodified SP's [Choo and Ranganathan, 2008]; frequency at position 2 of Gram-negative / eukaryotic SP's between brackets:
C (0.0% / 1.9%)
F (1.0% /2.2%)
H (0.3% /2.5%)
M (0.0% / 0.7%)
R (0.3% / 4.7%)
W (1.6% / 0.9%)
Y (0.3% / 2.5%)

This leaves the following 13 residues to be varied at position 2:
A (5.9% / 3.7%)
D (17.3% / 8.0%)
E (16.9% / 8.8%)
G (6.5% / 5.0%)
I (2.9% / 3.7%)
K (1.6% / 5.0%)
L (1.6% / 4.6%)
N (5.9% / 4.3%)
P (10.8% / 15.8%)
Q (4.9% / 4.9%)
S (5.5% / 9.0%)
T (10.8% / 5.6%)
V (5.9% / 6.3%)

The above results in 90 x 13 = 1170 sequences to be analyzed.

The 1170 sequences were generated. All sequences were given a code of format P#XZ or E#XZ, where:
P = prokaryotic SP; E = eukaryotic SP
# = internal designation number
X = amino acid at VH position 1
Z = amino acid at VH position 2

For example, P1AD comprises the prokaryotic SP and the 20-residue VH sequence has the first VH encoded amino acid E changed to A and the second VH encoded amino acid V changed to D.

To study the effect of the first two varied VH residues on SP cleavage, all 1170 sequences were analyzed *in silico* using prediction tool SignalP 4.1, with parameters further provided herein.

For all 1170 sequences, the position of the SP cleavage site was correctly predicted. The D scores for the 1170 sequences were compared (**Figure 3**). It was observed that overall, the effect on SP cleavage of the identity of the residue at position 2 did not depend heavily on the identity of the residue at position 1. For example, relatively low D scores were seen with K at position 2, irrespective of the residue at position 1 being A, D, E, Q or S. Furthermore, highest scores were generally obtained for sequences with A at position 1.

The results in **Figure 3** were used as follows to define the optimal combination of residues at positions 1 and 2 for each of nine primers, as summarized in **Figure 4****.**

First, for each of the 18 combinations of SP and VH gene segments, the variants with the highest D score were identified. For example, for the 65 sequences of code P1XZ, the variant with AV at position 1+2 had the highest D score (0.907). Interestingly, the best variants of the sequences with a bacterial SP all had AV (or sometimes also AT) at position 1+2. Similarly, the best variants of the sequences with a eukaryotic SP all had AP (or sometimes also AV) at position 1+2.

It is understood that based on the teachings provided herein, a person of ordinary skill in the art could also identify separate primers for use with a prokaryotic or eukaryotic signal peptide.

Preferably separate primers are not needed for VH's to be used with a prokaryotic or eukaryotic SP, a consensus sequence at position 1+2 was determined for each of the 9 primers, which in combination with both SP's gives a higher score/frequency than the corresponding WT sequence. Based on the data, this consensus sequence was defined as AP:
Combined with the bacterial SP, AP at position 1+2 gives a D score that is 0.030 to 0.065 (average 0.040) higher than the WT sequence.
Combined with the eukaryotic SP, AP at position 1+2 gives a D score that is 0.002 to 0.005 (average 0.003) higher than the WT sequence.

A is the most frequent amino acid at position 1 when combined with unmodified bacterial (41.7%) and eukaryotic (13.5%) SP's (see **Figure 3**).

P is the third most frequent amino acid at position 2 when combined with unmodified bacterial SP's (10.8%) and the most frequent amino acid at position 2 when combined with unmodified eukaryotic SP's (15.8%; see **Figure 3**).

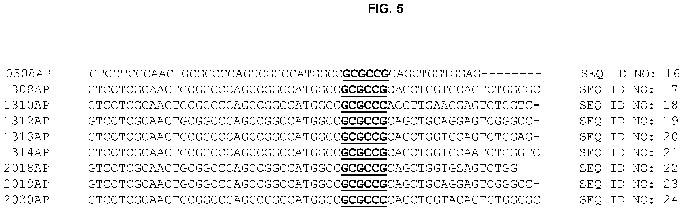

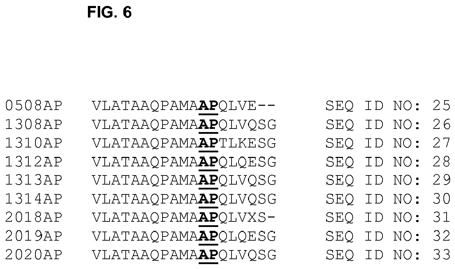

New FW (5') primers were designed that are the same as the primers listed in **Figure 5****,** except that in all of the new FW primers the first two VH codons were changed such that these encode AP instead of EV, EQ, Ql or QL (named 0508AP, 1308AP, 1310AP, etc.). Due to the degeneracy of the genetic code, four different codons exist for A (GCN) and P (CCN). For the primers, codons were chosen that are most homologous to the codons in the current primers (this varies per primer). Care was taken not to introduce new cloning sites3 of Sfil, BstEll and Xhol. The sequences of the resulting 9 new primers are given in **Figure 5**; protein translations of these primers are given in **Figure 6****.**

The designed primers were tested in parallel with current primers that do not modify the first and second amino acids encoded by the VH region. A summary of the results is as follows:

### Experimental results

### Primer design and analysis of amplification efficiency.

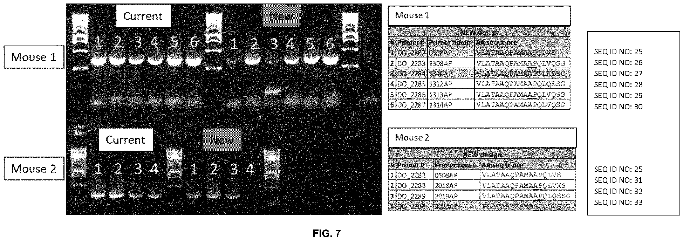

Two different exemplary MeMo® mouse lines were used. cDNA based on the nucleic acid within these mice was used for VH amplification with the new primers and current primers. The amplification efficiency was analyzed for each primer by comparing PCR product yields on agarose gel. see **Figure 7****.**

The following primers were shown to yield sufficient PCR product:
For IGHV1 family: 1308AP; 2018AP
For IGHV3 family: 0508AP; 2018AP
For IGVH4 family: 1312AP; 2019AP
For IGHV5 family: 1313AP
For IGHV7 family: 1314AP

The following primers produced low or no yield:
For IGHV1 family: 2020AP (no yield)
For IGHV2 family: 1310AP (low yield)

Three of new (AP) primers did not perform well in PCR to amplify VH gene segments from cDNA, therefore primer design was reconsidered. Poor results might be caused by sequence motifs such as long G/C stretches, which might cause undesirable secondary structures like dimers and hairpins. Inspection of used primer sequences showed that introduction of mutations resulted in relatively long G/C stretches of 10-11 bp, in part due to use of codon GCG for Ala and CCC/CCG for Pro. Although such stretches were also present in primers that did yield good results, any negative effect may depend on sequence properties elsewhere in primers, which varies per primer.

Degeneracy of genetic code allows to reduce length of G/C stretches by choosing other codons. Therefore, new primers were designed (version 2; AP2; named 0508AP2, 1310AP2, etc.) in which codons GCA and CCT were used for Ala and Pro, respectively. This was done not only for three primers that did not perform well, but also for other 6 primers.

To check in silico if version 2 / AP2 primers are expected to perform better, a subset was analyzed using Oligo Analyses feature of Vector NTI software. For example, for primer D0_2020 and its newer versions, following numbers of possible undesirable dimers and hairpins were predicted:
2020AP: 65 dimers and 26 hairpins (91 total). This is relatively high and may explain (in part) why this primer did not perform well.
2020AP2: 49 dimers and 19 hairpins (68 total). This is much less than for 2020AP. This suggests that 2020AP2 may perform better than 2020AP.

It was also checked whether further reduction of G/C content might lead to even fewer predicted secondary structures:
When Ala codon GCC that precedes VH was replaced by GCT or GCA in primer 2020AP2, 55 or 54 possible dimers and 22 possible hairpins were predicted (77 or 76 total). This is more than for 2020AP2, therefore this Ala codon was maintained as GCC.

Similar prediction results were obtained for other primers (not shown).

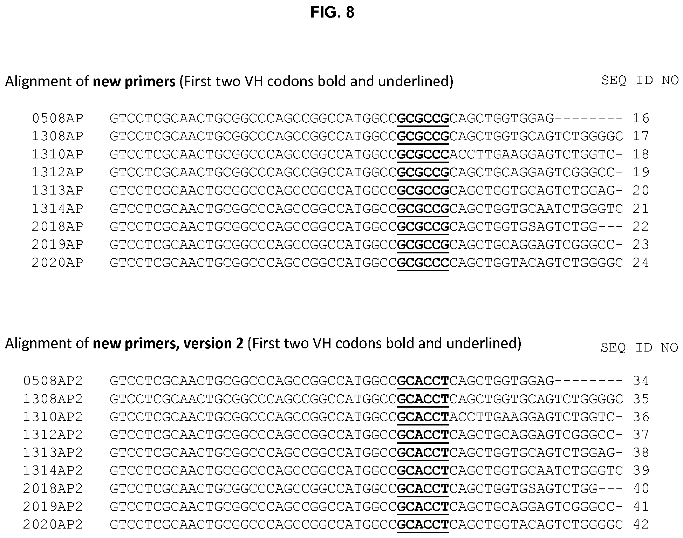

Alignments of the new (AP) and optimized (AP2) primers is shown in **Figure 8****.**

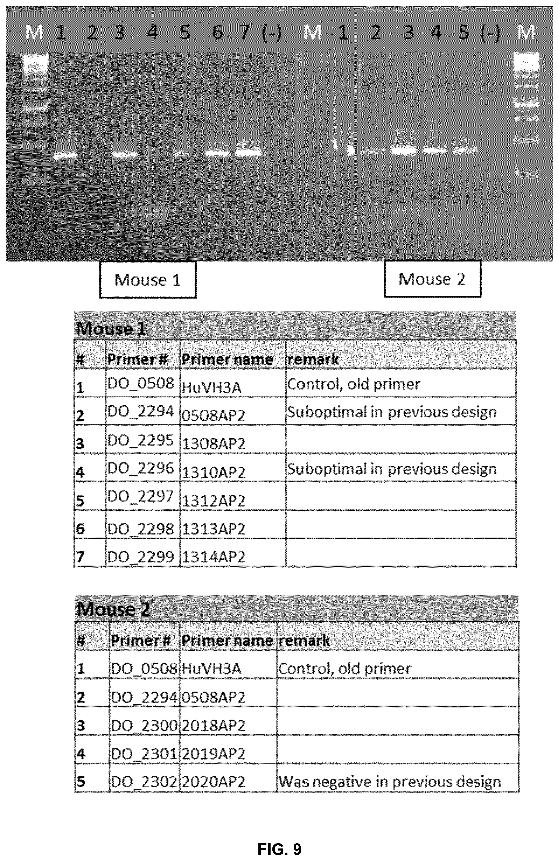

The majority of the AP2 primers were shown to yield sufficient PCR product. **See** **Figure 9****.** However, primers 0508AP2 and 1310AP2; (lanes # 2 & 4 for mouse 1) do not seem to show (much) improvement to previous design. Primer 2020AP2 (lane # 5 for mouse 2) works now whereas previous design (2020AP) did not. Negative controls (-) are negative.

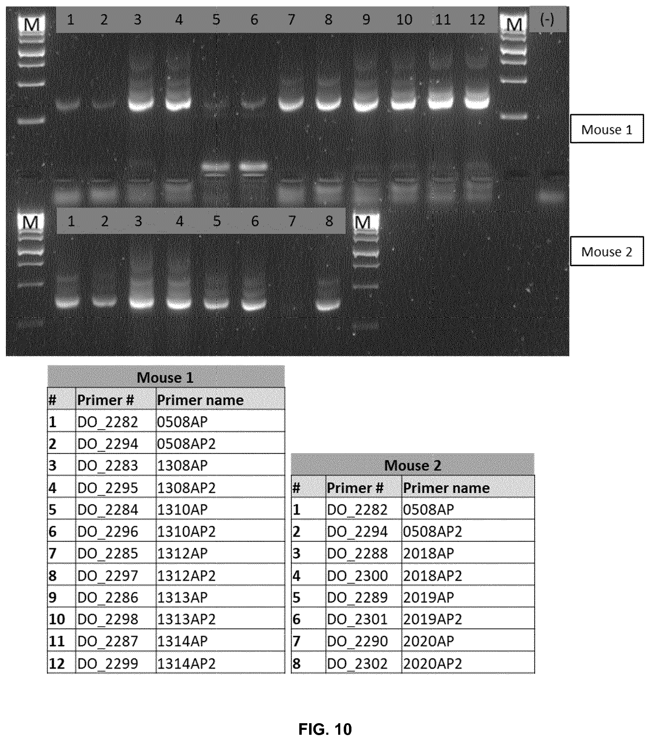

All new primers (AP and AP2) versions were tested in parallel to analyze amplification efficiency. See **Figure 10****.** As seen before, primer 2020AP2 (# 8 mouse 2) works, whereas 2020AP (# 7 mouse 2) does not. For both mice, primer 0508AP (# 1) seems to work bit better than 0508AP2 (# 2). In all other reactions, yields of AP2 are similar or slightly better compared to AP.

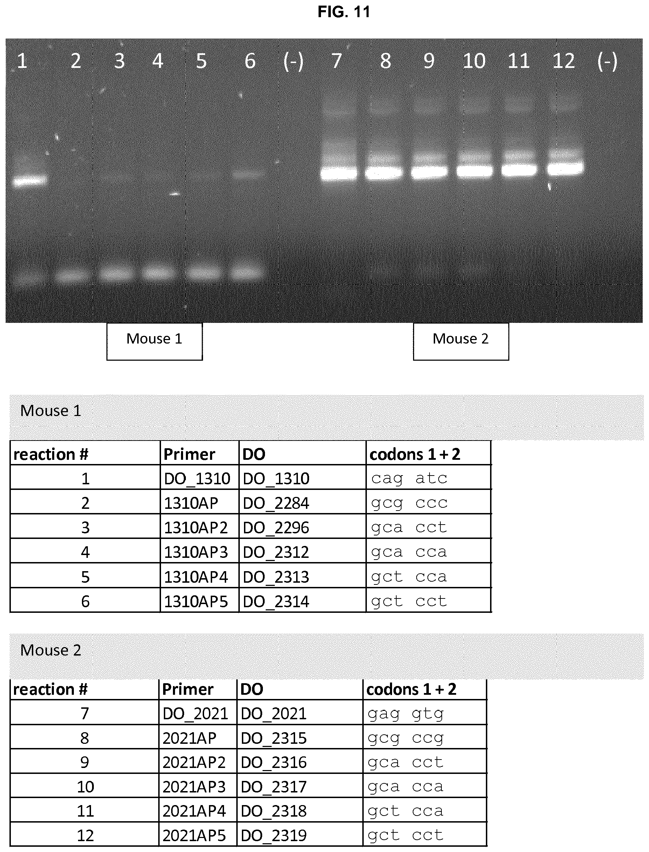

Further optimization of the 1310AP primer was initiated. In addition, an additional primer to the IGHV3 family was tested (2021AP). 5 different variants of the 1310AP primer were tested, together with five different variants of the 2021AP primer. **See** **Figure 11****.** New primer 1310AP5 (#6 mouse 1) gives clearly better results than primer 1310AP2 (#3 mouse 1) that previously performed best. New primers 2021AP to 2021AP5 (# 8 to 12) all perform similarly well.

Overall, the following primers are capable of yielding sufficient PCR product:
For IGHV1 family: 1308AP, 1308AP2, 2020AP2, 2018AP, 2018AP2
For IGHV2 family: 1310AP2, 1310AP3, 1310AP4, 1310AP5
For IGHV3 family: 0508AP, 0508AP2, 2018AP, 2018AP2, 2021AP, 2021AP2, 2021AP3, 2021AP4, 2021AP5
For IGVH4 family: 1312AP2 / 2019AP2
For IGHV5 family: 1313AP, 1313AP2
For IGHV6 family: 1310AP2, 1310AP3, 1310AP4, 1310AP5, 1312AP2/2019AP2
For IGHV7 family: 1314AP, 1314AP2

The PCR products of a variety of the above primers were purified and ligated into a vector for transformation into phage competent bacterial cells. Phage display libraries were generated. Colony PCR and sequencing was performed to determine insert frequency and sequence diversity.

### Analysis of VH diversity

Phage display libraries (size approximately 1E6-1E7) were built by cloning the amplified VH gene segments into a Fab-phage vector. Individual clones from these libraries were sequenced to determine the representation of the various VH gene families. The resulting sequences were analyzed to determine the representation of the various VH gene families. This representation (i.e. the percentage of each VH in the total number of amplified VH gene segments) was found to be similar for the new variant inducing primers and primers that amplify the non-varied VH sequences, demonstrating the primers that generate variants comprising the first two positions of the VH sequences do not affect the representation of the corresponding V gene segments and VH families in the phage library produced.

### Analysis of Fab expression

Individual clones from the libraries were used to produce non-purified periplasmic extracts containing soluble Fabs. Concentrations of these Fabs were determined using Octet quantitation. Most productions were found to have Fab yields in same range (approximately 10-15 µg/ml) (data not shown). Fabs with AP mutation produced well and overall resulted in higher average yields than WT Fabs (11.4 vs 10.0 µg/ml) demonstrating the utility of the variant generating primers and variation in the N-terminal variable regions.

### Analysis of Fab integrity

A subset of the produced soluble Fabs were subjected to SDS-PAGE and Western blotting. Bands of the expected sizes were visible on the resulting blots (data not shown).

### Summary of results

The experiments showed that the primers of an invention described herein can be used to generate phage display libraries with a diverse VH repertoire, and that Fabs can be expressed by members from these libraries. The new primers may be used to amplify cDNA encoding VH gene segments across the whole human variable region gene segment repertoire, whilst modifying the N-terminus of the encoded variable domain to prevent N-terminal pyroglutamic acid formation and/or increase Fab expression.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent, or similar purpose, unless expressly stated otherwise. Thus, unless expressly stated otherwise, each feature disclosed is one example only of a generic series of equivalent or similar features.

The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

### References

Choo, K. H., and Ranganathan, S. (2008). Flanking signal and mature peptide residues influence signal peptide cleavage. BMC Bioinformatics 9 Suppl 12, S15.

Fowler, E., Moyer, M., Krishna, R. G., Chin, C. C. Q., and Wold, F. (1996). Removal of N-terminal blocking groups from proteins, Current Protocols in Protein Science*.*

Jefferis, R. (2016). Review article. Posttranslational modifications and the immunogenicity of biotherapeutics. Journal of Immunology Research 2016*.*

Liu YD, Goetze AM, Bass RB, Flynn GC (2011). N-terminal glutamate to pyroglutamate conversion in vivo for human IgG2 antibodies. J Biol Chem. 2011 Apr 1;286(13):11211-7.

Petersen, T. N., Brunak, S., von Heijne, G., and Nielsen, H. (2011). SignalP 4.0: discriminating signal peptides from transmembrane regions. Nature Methods 8, 785-786.

Yu L., Vizel A., Huff M.B., Young M., Remmele R.L. Jr, He B. (2006). Investigation of N-terminal glutamate cyclization of recombinant monoclonal antibody in formulation development. J. Pharm. Biomed. Anal. 42(4): 455-63.

Ambrogelly A., Gozo S., Katiyar A., Dellatore S., Kune Y., Bhat R., Sun J., Li N., Wang D., Nowak C., Neill A., Ponniah G., King C., Mason B., Beck A, Liu H. (2018). Analytical comparability of recombinant monoclonal antibody therapeutics. MAbs 10(4): 513-538

## Claims

1. A polypeptide comprising a human immunoglobulin heavy chain variable domain, wherein the variable domain comprises an N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine.

2. The polypeptide of claim 1, wherein the human immunoglobulin heavy chain variable domain comprises an N-terminal alanine, optionally wherein the human immunoglobulin heavy chain variable domain comprises the N-terminal sequence alanine-proline.

3. The polypeptide of claim 1 or 2, wherein the polypeptide comprises a signal peptide upstream of the N-terminal amino acid of the human immunoglobulin heavy chain variable domain.

4. An antibody, antibody variant or antibody fragment comprising a polypeptide according to claim 1 or 2.

5. A nucleic acid encoding a polypeptide, antibody, antibody variant or antibody fragment according to any one of claims 1 to 4, optionally wherein the nucleic acid is within a vector or a phage.

6. A library comprising at least about 10⁶ distinct nucleic acids, vectors, or phages according to claim 5.

7. A method of simultaneously amplifying and modifying a nucleic acid that encodes a human immunoglobulin heavy chain variable domain, the method comprising:
(a) providing a nucleic acid that encodes a human immunoglobulin heavy chain variable domain; and
(b) performing a polymerase chain reaction with at least one 5' primer, at least one 3' primer and the nucleic acid to generate an amplified nucleic acid,
wherein the at least one 5' primer comprises a nucleic acid with a modification site that introduces a modification in the amplified nucleic acid such that the amplified nucleic acid encodes a human immunoglobulin heavy chain variable domain comprising an N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine.

8. The method of claim 7, wherein each amplified nucleic acid encodes a human immunoglobulin heavy chain variable domain comprising an N-terminal alanine, optionally wherein each amplified nucleic acid encodes a human immunoglobulin heavy chain variable domain comprising the N-terminal sequence alanine-proline.

9. The method of claim 7 or 8, wherein the at least one 5' primer encodes a signal peptide or portion of a signal peptide upstream of the modification site.

10. The method of any one of claims 7 to 9, wherein the nucleic acid(s) in step (a) is cDNA, optionally wherein the method comprises a prior step of extracting nucleic acids from B cells of an animal and generating cDNA from the nucleic acids to generate the nucleic acid(s) provided in step (a); further optionally wherein
i) the animal has been immunized with an antigen of interest, and the nucleic acids from the B cells encode heavy chains having specificity and affinity for the antigen of interest; and/or
ii) the animal is a transgenic murine animal comprising a human immunoglobulin heavy chain locus; and/or
iii) the animal is a transgenic murine animal comprising a common light chain.

11. The method of any of claims 7 to 10, wherein step (a) comprises providing a plurality of distinct nucleic acid encoded by at least one recombined human gene segment selected from each of the following human gene families: IGHV1, IGHV2, IGHV3, IGHV4, IGHV5, IGHV6 and IGHV7.

12. The method of any one of claims 7 to 11, wherein the method comprises:
(a) amplifying and modifying a nucleic acid encoded by an IGHV1 family gene using a 5' primer that is selected from 1308AP, 1308AP2, 2020AP2, 2018AP or 2018AP2; and/or
(b) amplifying and modifying a nucleic acid encoded by an IGHV2 family gene using a 5' primer that is selected from 1310AP2, 1310AP3, 1310AP4, or 1310AP5; and/or
(c) amplifying and modifying a nucleic acid encoded by an IGHV3 family gene using a 5' primer that is selected from 0508AP, 0508AP2, 2018AP, 2018AP2, 2021AP, 2021AP2, 2021AP3, 2021AP4, or2021AP5; and/or
(d) amplifying and modifying a nucleic acid encoded by an IGHV4 family gene using a 5' primer that is 1312AP; and/or
(e) amplifying and modifying a nucleic acid encoded by an IGHV5 family gene using a 5' primer that is selected from 1313AP, or 1313AP2;
(f) amplifying and modifying a nucleic acid encoded by an IGHV6 family gene using a 5' primer that is selected from 1310AP2, 1310AP3, 1310AP4, 1310AP5 or 1312AP2; and/or
(g) amplifying and modifying a nucleic acid encoded by an IGHV7 family gene using a 5' primer that is selected from 1314AP, or 1314AP2.

13. A 5' primer for amplifying and modifying any nucleic acid that encodes a human immunoglobulin heavy chain variable domain selected from one or more of the following human VH gene families: IGHV1, IGHV2, IGHV3, IGHV4, IGHV5, IGHV6 and IGHV7, wherein the primer comprises a modification site that introduces a modification in the amplified nucleic acid such that the amplified nucleic acid encodes a human immunoglobulin heavy chain variable domain comprising an N-terminal amino acid selected from the group consisting of: alanine, arginine, asparagine, aspartate, glycine, histidine, isoleucine, leucine, lysine, methionine, phenylalanine, proline, serine, threonine, tryptophan, tyrosine and valine.

14. The primer of claim 13, wherein the modification site is such that the amplified nucleic acid encodes a human immunoglobulin heavy chain variable domain comprising an N-terminal alanine, optionally wherein the modification site is such that the amplified nucleic acid encodes a human immunoglobulin heavy chain variable domain comprising an N-terminal alanine-proline.

15. A kit comprising at least one 5' primer selected from each of the following groups:
(a) 1308AP, 1308AP2, 2020AP2, 2018AP or 2018AP2;
(b) 1310AP2, 1310AP3, 1310AP4 or 1310AP5; a
(c) 0508AP, 0508AP2, 2018AP, 2018AP2, 2021AP, 2021AP2, 2021AP3, 2021AP4, or 2021AP5;
(d) 1312AP2;
(e) 1313AP or 1313AP2;
(f) 1310AP2, 1310AP3, 1310AP4, 1310AP5, or 1312AP2;
and
(g) 1314AP2 or 1314AP.


SEQUENCE LISTING

<110>  Merus N.V.

<120>  Modified human variable domains

<130>  P274251EP

<160>  168

<170>  PatentIn version 3.5

<210>  1
<211>  22
<212>  PRT
<213>  Artificial Sequence

<220>
<223>  signal peptide that can be used for VH domain expression in
       prokaryotic cells

<400>  1

Met Lys Tyr Leu Leu Pro Thr Ala Ala Ala Gly Leu Leu Leu Leu Ala
1               5                   10                  15

Ala Gln Pro Ala Met Ala
            20

<210>  2
<211>  8
<212>  PRT
<213>  Artificial Sequence

<220>
<223>  VH domain

<400>  2

Gln Val Gln Leu Val Gln Ser Gly
1               5

<210>  3
<211>  30
<212>  PRT
<213>  Artificial Sequence

<220>
<223>  signal peptide + VH domain

<400>  3

Met Lys Tyr Leu Leu Pro Thr Ala Ala Ala Gly Leu Leu Leu Leu Ala
1               5                   10                  15

Ala Gln Pro Ala Met Ala Gln Val Gln Leu Val Gln Ser Gly
            20                  25                  30

<210>  4
<211>  20
<212>  PRT
<213>  Artificial Sequence

<220>
<223>   signal peptide that can be used for VH domain expression in
       eukaryotic cells

<400>  4

Met Gly Trp Ser Cys Ile Ile Leu Phe Leu Val Leu Leu Leu Ala Gln
1               5                   10                  15


Pro Ala Met Ala
            20


<210>  5
<211>  6
<212>  PRT
<213>  Artificial Sequence

<220>
<223>  sequence at the C-terminal end of the signal peptide

<400>  5

Ala Gln Pro Ala Met Ala
1               5


<210>  6
<211>  7
<212>  PRT
<213>  Artificial Sequence

<220>
<223>  residues of the VH domain and signal peptide that flank the
       signal peptide cleavage site

<400>  6

Ala Gln Pro Ala Met Ala Ala
1               5


<210>  7
<211>  8
<212>  PRT
<213>  Artificial Sequence

<220>
<223>  residues of the VH domain and signal peptide that flank the
       signal peptide cleavage site

<400>  7

Ala Gln Pro Ala Met Ala Ala Pro
1               5

<210>  8
<211>  23
<212>  PRT
<213>  Artificial Sequence

<220>
<223>  amino acid residues of the VH domain and signal peptide that
      flank the signal peptide cleavage site

<400>  8

Met Lys Tyr Leu Leu Pro Thr Ala Ala Ala Gly Leu Leu Leu Leu Ala
1               5                   10                  15

Ala Gln Pro Ala Met Ala Ala
            20

<210>  9
<211>  24
<212>  PRT
<213>  Artificial Sequence

<220>
<223>  amino acid residues of the VH domain and signal peptide that
      flank the signal peptide cleavage site

<400>  9

Met Lys Tyr Leu Leu Pro Thr Ala Ala Ala Gly Leu Leu Leu Leu Ala
1               5                   10                  15

Ala Gln Pro Ala Met Ala Ala Pro
            20

<210>  10
<211>  21
<212>  PRT
<213>  Artificial Sequence

<220>
<223>  amino acid residues of the VH domain and signal peptide that
      flank the signal peptide cleavage site

<400>  10

Met Gly Trp Ser Cys Ile Ile Leu Phe Leu Val Leu Leu Leu Ala Gln
1               5                   10                  15

Pro Ala Met Ala Ala
            20

<210>  11
<211>  22
<212>  PRT
<213>  Artificial Sequence

<220>

<223>  amino acid residues of the VH domain and signal peptide that
       flank the signal peptide cleavage site

<400>  11

Met Gly Trp Ser Cys Ile Ile Leu Phe Leu Val Leu Leu Leu Ala Gln
1               5                   10                  15

Pro Ala Met Ala Ala Pro
            20

<210>  12
<211>  32
<212>  DNA
<213>  Artificial Sequence

<220>
<223>  HuJH1/2xho primer

<400>  12
tattgttacc tcgagacggt gaccagggtg cc                                32

<210>  13
<211>  32
<212>  DNA
<213>  Artificial Sequence

<220>
<223>  HuJH3xho primer

<400>  13
tattgttacc tcgagacggt gaccattgtc cc                                32

<210>  14
<211>  32
<212>  DNA
<213>  Artificial Sequence

<220>
<223>  HuJH4/5xho primer

<400>  14
tattgttacc tcgagacggt gaccagggtt cc                                32

<210>  15
<211>  32
<212>  DNA
<213>  Artificial Sequence

<220>
<223>  HuJH6xho primer

<400>  15
tattgttacc tcgagacggt gaccgtggtc cc                                32

<210>  16
<211>  51

<212>  DNA
<213>  Artificial Sequence

<220>
<223>  0508AP primer

<400>  16
gtcctcgcaa ctgcggccca gccggccatg gccgcgccgc agctggtgga g          51


<210>  17
<211>  59
<212>  DNA
<213>  Artificial Sequence

<220>
<223>  1308AP primer

<400>  17
gtcctcgcaa ctgcggccca gccggccatg gccgcgccgc agctggtgca gtctggggc  59


<210>  18
<211>  58
<212>  DNA
<213>  Artificial Sequence

<220>
<223>  1310AP primer

<400>  18
gtcctcgcaa ctgcggccca gccggccatg gccgcgccca ccttgaagga gtctggtc   58


<210>  19
<211>  58
<212>  DNA
<213>  Artificial Sequence

<220>
<223>  1312AP primer

<400>  19
gtcctcgcaa ctgcggccca gccggccatg gccgcgccgc agctgcagga gtcgggcc   58


<210>  20
<211>  58
<212>  DNA
<213>  Artificial Sequence

<220>
<223>  1313AP primer

<400>  20
gtcctcgcaa ctgcggccca gccggccatg gccgcgccgc agctggtgca gtctggag   58


<210>  21
<211>  59
<212>  DNA
<213>  Artificial Sequence

<220>
<223>  1314AP primer

<400>  21
gtcctcgcaa ctgcggccca gccggccatg gccgcgccgc agctggtgca atctgggtc        59


<210>  22
<211>  56
<212>  DNA
<213>  Artificial Sequence

<220>
<223>  2018AP primer

<400>  22
gtcctcgcaa ctgcggccca gccggccatg gccgcgccgc agctggtgsa gtctgg           56


<210>  23
<211>  58
<212>  DNA
<213>  Artificial Sequence

<220>
<223>  2019AP primer

<400>  23
gtcctcgcaa ctgcggccca gccggccatg gccgcgccgc agctgcagga gtcgggcc         58


<210>  24
<211>  59
<212>  DNA
<213>  Artificial Sequence

<220>
<223>  2020AP primer

<400>  24
gtcctcgcaa ctgcggccca gccggccatg gccgcgcccc agctggtaca gtctggggc        59


<210>  25
<211>  17
<212>  PRT
<213>  Artificial Sequence

<220>
<223>  protein translation of 0508AP primer

<400>  25

Val Leu Ala Thr Ala Ala Gln Pro Ala Met Ala Ala Pro Gln Leu Val
1               5                   10                  15


Glu



<210>  26
<211>  19

<212>  PRT
<213>  Artificial Sequence

<220>
<223>  protein translation of 1308AP primer

<400>  26

Val Leu Ala Thr Ala Ala Gln Pro Ala Met Ala Ala Pro Gln Leu Val
1               5                   10                  15

Gln Ser Gly

<210>  27
<211>  19
<212>  PRT
<213>  Artificial Sequence

<220>
<223>  protein translation of 1310AP primer

<400>  27

Val Leu Ala Thr Ala Ala Gln Pro Ala Met Ala Ala Pro Thr Leu Lys
1               5                   10                  15

Glu Ser Gly

<210>  28
<211>  19
<212>  PRT
<213>  Artificial Sequence

<220>
<223>  protein translation of 1312AP primer

<400>  28

Val Leu Ala Thr Ala Ala Gln Pro Ala Met Ala Ala Pro Gln Leu Gln
1               5                   10                  15

Glu Ser Gly

<210>  29
<211>  19
<212>  PRT
<213>  Artificial Sequence

<220>
<223>  protein translation of 1313AP primer

<400>  29

Val Leu Ala Thr Ala Ala Gln Pro Ala Met Ala Ala Pro Gln Leu Val

1               5                   10                  15

Gln Ser Gly

<210>  30
<211>  19
<212>  PRT
<213>  Artificial Sequence

<220>
<223>  protein translation of 1314AP primer

<400>  30

Val Leu Ala Thr Ala Ala Gln Pro Ala Met Ala Ala Pro Gln Leu Val
1               5                   10                  15

Gln Ser Gly

<210>  31
<211>  18
<212>  PRT
<213>  Artificial Sequence

<220>
<223>  protein translation of 2018AP primer

<220>
<221>  misc_feature
<222>  (17)..(17)
<223>  Xaa can be any naturally occurring amino acid

<400>  31

Val Leu Ala Thr Ala Ala Gln Pro Ala Met Ala Ala Pro Gln Leu Val
1               5                   10                  15

Xaa Ser

<210>  32
<211>  19
<212>  PRT
<213>  Artificial Sequence

<220>
<223>  protein translation of 2019AP primer

<400>  32

Val Leu Ala Thr Ala Ala Gln Pro Ala Met Ala Ala Pro Gln Leu Gln
1               5                   10                  15

Glu Ser Gly

<210> 33
<211> 19
<212> PRT
<213> Artificial Sequence

<220>
<223> protein translation of 2020AP primer

<400> 33

Val Leu Ala Thr Ala Ala Gln Pro Ala Met Ala Ala Pro Gln Leu Val
1               5                   10                  15

Gln Ser Gly

<210> 34
<211> 51
<212> DNA
<213> Artificial Sequence

<220>
<223> 0508AP2 primer

<400> 34
gtcctcgcaa ctgcggccca gccggccatg gccgcacctc agctggtgga g 51

<210> 35
<211> 59
<212> DNA
<213> Artificial Sequence

<220>
<223> 1308AP2 primer

<400> 35
gtcctcgcaa ctgcggccca gccggccatg gccgcacctc agctggtgca gtctggggc 59

<210> 36
<211> 58
<212> DNA
<213> Artificial Sequence

<220>
<223> 1310AP2 primer

<400> 36
gtcctcgcaa ctgcggccca gccggccatg gccgcaccta ccttgaagga gtctggtc 58

<210> 37
<211> 58
<212> DNA
<213> Artificial Sequence

<220>
<223>  1312AP2 primer

<400>  37
gtcctcgcaa ctgcggccca gccggccatg gccgcacctc agctgcagga gtcgggcc        58


<210>  38
<211>  58
<212>  DNA
<213>  Artificial Sequence

<220>
<223>  1313AP2 primer

<400>  38
gtcctcgcaa ctgcggccca gccggccatg gccgcacctc agctggtgca gtctggag        58


<210>  39
<211>  59
<212>  DNA
<213>  Artificial Sequence

<220>
<223>  1314AP2 primer

<400>  39
gtcctcgcaa ctgcggccca gccggccatg gccgcacctc agctggtgca atctgggtc       59


<210>  40
<211>  56
<212>  DNA
<213>  Artificial Sequence

<220>
<223>  2018AP2 primer

<400>  40
gtcctcgcaa ctgcggccca gccggccatg gccgcacctc agctggtgsa gtctgg          56


<210>  41
<211>  58
<212>  DNA
<213>  Artificial Sequence

<220>
<223>  2019AP2 primer

<400>  41
gtcctcgcaa ctgcggccca gccggccatg gccgcacctc agctgcagga gtcgggcc        58


<210>  42
<211>  59
<212>  DNA
<213>  Artificial Sequence

<220>
<223>  2020AP2 primer

<400>  42
gtcctcgcaa ctgcggccca gccggccatg gccgcacctc agctggtaca gtctggggc        59


<210>  43
<211>  26
<212>  DNA
<213>  Homo sapiens

<400>  43
caggtgcagc tggtgcagtc tggggc                                            26


<210>  44
<211>  26
<212>  DNA
<213>  Homo sapiens

<400>  44
caggtccagc ttgtgcagtc tggggc                                            26


<210>  45
<211>  26
<212>  DNA
<213>  Homo sapiens

<400>  45
caggtgcagc tggtgcagtc tggggc                                            26


<210>  46
<211>  26
<212>  DNA
<213>  Homo sapiens

<400>  46
caggttcagc tggtgcagtc tggagc                                            26


<210>  47
<211>  26
<212>  DNA
<213>  Homo sapiens

<400>  47
caggtccagc tggtacagtc tggggc                                            26


<210>  48
<211>  26
<212>  DNA
<213>  Homo sapiens

<400>  48
cagatgcagc tggtgcagtc tggggc                                            26


<210>  49
<211>  26
<212>  DNA
<213>  Homo sapiens

<400>  49
caggtgcagc tggtgcagtc tggggc                                          26


<210>  50
<211>  26
<212>  DNA
<213>  Homo sapiens

<400>  50
caaatgcagc tggtgcagtc tgggcc                                          26


<210>  51
<211>  26
<212>  DNA
<213>  Homo sapiens

<400>  51
caggtgcagc tggtgcagtc tggggc                                          26


<210>  52
<211>  26
<212>  DNA
<213>  Homo sapiens

<400>  52
gaggtccagc tggtacagtc tggggc                                          26


<210>  53
<211>  26
<212>  DNA
<213>  Homo sapiens

<400>  53
caggtgcagc tggtgcagtc tggggc                                          26


<210>  54
<211>  26
<212>  DNA
<213>  Artificial Sequence

<220>
<223>  consensus sequence - Fig.12

<400>  54
caggtgcagc tggtgcagtc tggggc                                          26


<210>  55
<211>  23
<212>  DNA
<213>  Homo sapiens

<400>  55
caggtgcagc tggtgcagtc tgg                                             23


<210>  56
<211>  23

<212>  DNA
<213>  Homo sapiens

<400>  56
caggtccagc ttgtgcagtc tgg                                                23


<210>  57
<211>  23
<212>  DNA
<213>  Homo sapiens

<400>  57
caggtgcagc tggtgcagtc tgg                                                23


<210>  58
<211>  23
<212>  DNA
<213>  Homo sapiens

<400>  58
caggttcagc tggtgcagtc tgg                                                23


<210>  59
<211>  23
<212>  DNA
<213>  Homo sapiens

<400>  59
caggtccagc tggtacagtc tgg                                                23


<210>  60
<211>  23
<212>  DNA
<213>  Homo sapiens

<400>  60
cagatgcagc tggtgcagtc tgg                                                23


<210>  61
<211>  23
<212>  DNA
<213>  Homo sapiens

<400>  61
caggtgcagc tggtgcagtc tgg                                                23


<210>  62
<211>  23
<212>  DNA
<213>  Homo sapiens

<400>  62
caaatgcagc tggtgcagtc tgg                                                23


<210>  63
<211>  23

<212>  DNA
<213>  Homo sapiens

<400>  63
caggtgcagc tggtgcagtc tgg                                                23


<210>  64
<211>  23
<212>  DNA
<213>  Homo sapiens

<400>  64
gaggtccagc tggtacagtc tgg                                                23


<210>  65
<211>  23
<212>  DNA
<213>  Homo sapiens

<400>  65
caggtgcagc tggtgcagtc tgg                                                23


<210>  66
<211>  23
<212>  DNA
<213>  Artificial Sequence

<220>
<223>  consensus sequence - Fig.14

<400>  66
caggtgcagc tggtgcagtc tgg                                                23


<210>  67
<211>  58
<212>  DNA
<213>  Artificial Sequence

<220>
<223>  1310AP5

<400>  67
gtcctcgcaa ctgcggccca gccggccatg gccgctccta ccttgaagga gtctggtc          58


<210>  68
<211>  25
<212>  DNA
<213>  Homo sapiens

<400>  68
cagatcacct tgaaggagtc tggtc                                              25


<210>  69
<211>  25
<212>  DNA
<213>  Homo sapiens

<400>  69
caggtcacct tgaaggagtc tggtc                                          25


<210>  70
<211>  25
<212>  DNA
<213>  Homo sapiens

<400>  70
caggtcacct tgagggagtc tggtc                                          25


<210>  71
<211>  25
<212>  DNA
<213>  Homo sapiens

<400>  71
caggtcacct tgaaggagtc tggtc                                          25


<210>  72
<211>  25
<212>  DNA
<213>  Artificial Sequence

<220>
<223>  consensus sequence - Fig. 16

<400>  72
caggtcacct tgaaggagtc tggtc                                          25


<210>  73
<211>  18
<212>  DNA
<213>  Homo sapiens

<400>  73
caggtgcagc tggtggag                                                  18


<210>  74
<211>  18
<212>  DNA
<213>  Homo sapiens

<400>  74
caggtgcagc tggtggag                                                  18


<210>  75
<211>  18
<212>  DNA
<213>  Homo sapiens

<400>  75
caggtgcagc tggtggag                                                  18


<210>  76
<211>  18

<212>  DNA
<213>  Homo sapiens

<400>  76
caggtgcagc tggtggag                                                    18


<210>  77
<211>  18
<212>  DNA
<213>  Homo sapiens

<400>  77
caggtgcagc tggtggag                                                    18


<210>  78
<211>  18
<212>  DNA
<213>  Homo sapiens

<400>  78
caggtgcagc tggtggag                                                    18


<210>  79
<211>  18
<212>  DNA
<213>  Homo sapiens

<400>  79
gaggtgcagc tggtggag                                                    18


<210>  80
<211>  18
<212>  DNA
<213>  Homo sapiens

<400>  80
gaggtgcagc tggtggag                                                    18


<210>  81
<211>  18
<212>  DNA
<213>  Homo sapiens

<400>  81
gaggtgcagc tggtggag                                                    18


<210>  82
<211>  18
<212>  DNA
<213>  Homo sapiens

<400>  82
gaggtgcagc tggtggag                                                    18


<210>  83
<211>  18

<212>  DNA
<213>  Homo sapiens

<400>  83
gaggtgcagc tgttggag                                                    18


<210>  84
<211>  18
<212>  DNA
<213>  Homo sapiens

<400>  84
gaggtgcagc tgttggag                                                    18


<210>  85
<211>  18
<212>  DNA
<213>  Homo sapiens

<400>  85
gaagtgcagc tggtggag                                                    18


<210>  86
<211>  18
<212>  DNA
<213>  Homo sapiens

<400>  86
gaagtgcagc tggtggag                                                    18


<210>  87
<211>  18
<212>  DNA
<213>  Homo sapiens

<400>  87
gaagtgcagc tggtggag                                                    18


<210>  88
<211>  18
<212>  DNA
<213>  Homo sapiens

<400>  88
gaggtgcagc tggtggag                                                    18


<210>  89
<211>  18
<212>  DNA
<213>  Homo sapiens

<400>  89
gaggtgcagc tggtggag                                                    18


<210>  90
<211>  18

<212>  DNA
<213>  Homo sapiens

<400>  90
gaggtgcagc tggtggag                                                    18


<210>  91
<211>  18
<212>  DNA
<213>  Homo sapiens

<400>  91
gaggtgcagc tggtggag                                                    18


<210>  92
<211>  18
<212>  DNA
<213>  Homo sapiens

<400>  92
gaggtgcagc tggtggag                                                    18


<210>  93
<211>  18
<212>  DNA
<213>  Homo sapiens

<400>  93
gaggtgcagc tggtggag                                                    18


<210>  94
<211>  18
<212>  DNA
<213>  Homo sapiens

<400>  94
gaggtgcagc tggtggag                                                    18


<210>  95
<211>  18
<212>  DNA
<213>  Homo sapiens

<400>  95
gaggtgcagc tggtggag                                                    18


<210>  96
<211>  18
<212>  DNA
<213>  Homo sapiens

<400>  96
gaggtgcagc tggtggag                                                    18


<210>  97
<211>  18

<212>  DNA
<213>  Homo sapiens

<400>  97
gaggtgcagc tggtggag                                                    18


<210>  98
<211>  18
<212>  DNA
<213>  Artificial Sequence

<220>
<223>  consensus sequence - Fig.17

<400>  98
gaggtgcagc tggtggag                                                    18


<210>  99
<211>  58
<212>  DNA
<213>  Artificial Sequence

<220>
<223>  2021AP2

<400>  99
gtcctcgcaa ctgcggccca gccggccatg gccgcacctc agctgttgga gtctgggg        58


<210>  100
<211>  25
<212>  DNA
<213>  Homo sapiens

<400>  100
gaggtgcagc tggtggagtc tgggg                                            25


<210>  101
<211>  25
<212>  DNA
<213>  Homo sapiens

<400>  101
gaagtgcagc tggtggagtc tgggg                                            25


<210>  102
<211>  25
<212>  DNA
<213>  Homo sapiens

<400>  102
caggtgcagc tggtggagtc tgggg                                            25


<210>  103
<211>  25
<212>  DNA
<213>  Homo sapiens

<400>  103
gaggtgcagc tggtggagtc tgggg                                              25


<210>  104
<211>  25
<212>  DNA
<213>  Homo sapiens

<400>  104
gaggtgcagc tggtggagtc tgggg                                              25


<210>  105
<211>  25
<212>  DNA
<213>  Homo sapiens

<400>  105
gaggtgcagc tggtggagtc tgggg                                              25


<210>  106
<211>  25
<212>  DNA
<213>  Homo sapiens

<400>  106
gaggtgcagc tggtggagtc tgggg                                              25


<210>  107
<211>  25
<212>  DNA
<213>  Homo sapiens

<400>  107
gaggtgcagc tgttggagtc tgggg                                              25


<210>  108
<211>  25
<212>  DNA
<213>  Homo sapiens

<400>  108
gaggtgcagc tgttggagtc tgggg                                              25


<210>  109
<211>  25
<212>  DNA
<213>  Homo sapiens

<400>  109
caggtgcagc tggtggagtc tgggg                                              25


<210>  110
<211>  25
<212>  DNA
<213>  Homo sapiens

<400>  110
caggtgcagc tggtggagtc tgggg                                              25


<210>  111
<211>  25
<212>  DNA
<213>  Homo sapiens

<400>  111
caggtgcagc tggtggagtc tgggg                                              25


<210>  112
<211>  25
<212>  DNA
<213>  Homo sapiens

<400>  112
caggtgcagc tggtggagtc tgggg                                              25


<210>  113
<211>  25
<212>  DNA
<213>  Homo sapiens

<400>  113
gaagtgcagc tggtggagtc tgggg                                              25


<210>  114
<211>  25
<212>  DNA
<213>  Homo sapiens

<400>  114
gaagtgcagc tggtggagtc tgggg                                              25


<210>  115
<211>  25
<212>  DNA
<213>  Homo sapiens

<400>  115
gaggtgcagc tggtggagtc tgggg                                              25


<210>  116
<211>  25
<212>  DNA
<213>  Homo sapiens

<400>  116
gaggtgcagc tggtggagtc tgggg                                              25


<210>  117
<211>  25
<212>  DNA
<213>  Homo sapiens

<400>  117
gaggtgcagc tggtggagtc tggag                                           25


<210>  118
<211>  25
<212>  DNA
<213>  Homo sapiens

<400>  118
gaggtgcagc tggtggagtc tgggg                                           25


<210>  119
<211>  25
<212>  DNA
<213>  Homo sapiens

<400>  119
gaggtgcagc tggtggagtc tgggg                                           25


<210>  120
<211>  25
<212>  DNA
<213>  Homo sapiens

<400>  120
gaggtgcagc tggtggagtc tgggg                                           25


<210>  121
<211>  25
<212>  DNA
<213>  Homo sapiens

<400>  121
gaggtgcagc tggtggagtc tgggg                                           25


<210>  122
<211>  25
<212>  DNA
<213>  Homo sapiens

<400>  122
gaggtgcagc tggtggagtc tgggg                                           25


<210>  123
<211>  25
<212>  DNA
<213>  Homo sapiens

<400>  123
gaggtgcagc tggtggagtc cgggg                                           25


<210>  124
<211>  25
<212>  DNA
<213>  Homo sapiens

<400>  124
caggtgcagc tggtggagtc tgggg                                              25


<210>  125
<211>  25
<212>  DNA
<213>  Artificial Sequence

<220>
<223>  consensus sequence - Fig.18

<400>  125
gaggtgcagc tggtggagtc tgggg                                              25


<210>  126
<211>  23
<212>  DNA
<213>  Homo sapiens

<400>  126
gaggtgcagc tggtggagtc tgg                                                23


<210>  127
<211>  23
<212>  DNA
<213>  Homo sapiens

<400>  127
gaagtgcagc tggtggagtc tgg                                                23


<210>  128
<211>  23
<212>  DNA
<213>  Homo sapiens

<400>  128
caggtgcagc tggtggagtc tgg                                                23


<210>  129
<211>  23
<212>  DNA
<213>  Homo sapiens

<400>  129
gaggtgcagc tggtggagtc tgg                                                23


<210>  130
<211>  23
<212>  DNA
<213>  Homo sapiens

<400>  130
gaggtgcagc tggtggagtc tgg                                                23


<210>  131
<211>  23

<212>  DNA
<213>  Homo sapiens

<400>  131
gaggtgcagc tggtggagtc tgg                                              23


<210>  132
<211>  23
<212>  DNA
<213>  Homo sapiens

<400>  132
gaggtgcagc tggtggagtc tgg                                              23


<210>  133
<211>  23
<212>  DNA
<213>  Homo sapiens

<400>  133
gaggtgcagc tgttggagtc tgg                                              23


<210>  134
<211>  23
<212>  DNA
<213>  Homo sapiens

<400>  134
gaggtgcagc tgttggagtc tgg                                              23


<210>  135
<211>  23
<212>  DNA
<213>  Homo sapiens

<400>  135
caggtgcagc tggtggagtc tgg                                              23


<210>  136
<211>  23
<212>  DNA
<213>  Homo sapiens

<400>  136
caggtgcagc tggtggagtc tgg                                              23


<210>  137
<211>  23
<212>  DNA
<213>  Homo sapiens

<400>  137
caggtgcagc tggtggagtc tgg                                              23


<210>  138
<211>  23

<212>  DNA
<213>  Homo sapiens

<400>  138
caggtgcagc tggtggagtc tgg                                              23


<210>  139
<211>  23
<212>  DNA
<213>  Homo sapiens

<400>  139
gaagtgcagc tggtggagtc tgg                                              23


<210>  140
<211>  23
<212>  DNA
<213>  Homo sapiens

<400>  140
gaagtgcagc tggtggagtc tgg                                              23


<210>  141
<211>  23
<212>  DNA
<213>  Homo sapiens

<400>  141
gaggtgcagc tggtggagtc tgg                                              23


<210>  142
<211>  23
<212>  DNA
<213>  Homo sapiens

<400>  142
gaggtgcagc tggtggagtc tgg                                              23


<210>  143
<211>  23
<212>  DNA
<213>  Homo sapiens

<400>  143
gaggtgcagc tggtggagtc tgg                                              23


<210>  144
<211>  23
<212>  DNA
<213>  Homo sapiens

<400>  144
gaggtgcagc tggtggagtc tgg                                              23


<210>  145
<211>  23

<212>  DNA
<213>  Homo sapiens

<400>  145
gaggtgcagc tggtggagtc tgg                                               23


<210>  146
<211>  23
<212>  DNA
<213>  Homo sapiens

<400>  146
gaggtgcagc tggtggagtc tgg                                               23


<210>  147
<211>  23
<212>  DNA
<213>  Homo sapiens

<400>  147
gaggtgcagc tggtggagtc tgg                                               23


<210>  148
<211>  23
<212>  DNA
<213>  Homo sapiens

<400>  148
gaggtgcagc tggtggagtc tgg                                               23


<210>  149
<211>  23
<212>  DNA
<213>  Homo sapiens

<400>  149
gaggtgcagc tggtggagtc cgg                                               23


<210>  150
<211>  23
<212>  DNA
<213>  Homo sapiens

<400>  150
caggtgcagc tggtggagtc tgg                                               23


<210>  151
<211>  23
<212>  DNA
<213>  Artificial Sequence

<220>
<223>  consensus sequence - Fig.19

<400>  151
gaggtgcagc tggtggagtc tgg                                               23

<210>  152
<211>  25
<212>  DNA
<213>  Homo sapiens

<400>  152
caggtgcagc tgcaggagtc gggcc                                          25


<210>  153
<211>  25
<212>  DNA
<213>  Homo sapiens

<400>  153
caggtgcagc tgcaggagtc gggcc                                          25


<210>  154
<211>  25
<212>  DNA
<213>  Homo sapiens

<400>  154
cagctgcagc tgcaggagtc cggct                                          25


<210>  155
<211>  25
<212>  DNA
<213>  Homo sapiens

<400>  155
caggtgcagc tgcaggagtc gggcc                                          25


<210>  156
<211>  25
<212>  DNA
<213>  Homo sapiens

<400>  156
caggtgcagc tgcaggagtc gggcc                                          25


<210>  157
<211>  25
<212>  DNA
<213>  Homo sapiens

<400>  157
caggtgcagc tacagcagtg gggcg                                          25


<210>  158
<211>  25
<212>  DNA
<213>  Homo sapiens

<400>  158
caggtgcagc tgcaggagtc gggcc                                          25

<210>  159
<211>  25
<212>  DNA
<213>  Homo sapiens

<400>  159
cagctgcagc tgcaggagtc gggcc                                          25


<210>  160
<211>  25
<212>  DNA
<213>  Homo sapiens

<400>  160
caggtgcagc tgcaggagtc gggcc                                          25


<210>  161
<211>  25
<212>  DNA
<213>  Homo sapiens

<400>  161
caggtgcagc tgcaggagtc gggcc                                          25


<210>  162
<211>  25
<212>  DNA
<213>  Homo sapiens

<400>  162
caggtgcagc tgcaggagtc gggcc                                          25


<210>  163
<211>  25
<212>  DNA
<213>  Homo sapiens

<400>  163
gaagtgcagc tggtgcagtc tggag                                          25


<210>  164
<211>  25
<212>  DNA
<213>  Homo sapiens

<400>  164
gaggtgcagc tggtgcagtc tggag                                          25


<210>  165
<211>  25
<212>  DNA
<213>  Artificial Sequence

<220>
<223>  consensus sequence - Fig.23

<400>  165
gaagtgcagc tggtgcagtc tggag                                              25


<210>  166
<211>  26
<212>  DNA
<213>  Homo sapiens

<400>  166
caggtacagc tgcagcagtc aggtcc                                             26


<210>  167
<211>  26
<212>  DNA
<213>  Homo sapiens

<400>  167
caggtgcagc tggtgcaatc tgggtc                                             26


<210>  168
<211>  23
<212>  DNA
<213>  Artificial Sequence

<220>
<223>  consensus sequence - Fig. 25

<400>  168
catcagctgg tgcaatctgg gtc                                                23